# EUROPEAN PATENT APPLICATION

(11) **EP 2 433 939 A1**
(43) Date of publication of application: **28.03.2012**
(21) Application number: 10777998.5
(22) Date of filing: 30.04.2010
(51) Int. Cl.: C07D 471/04, A61K 31/437, A61K 9/48, A61K 9/08, A61P 25/18, A61P 25/22, A61P 25/28, A61P 25/24

(54) **SUBSTITUTED 8-SULFONYL-2,3,4,5-TETRAHYDRO-1H-GAMMA-CARBOLINES, LIGANDS AND PHARMACEUTICAL COMPOSITION; METHOD FOR THE PRODUCTION AND USE OF SAME**

(30) Priority: 22.05.2009 RU 2009119369
(71) Applicant: Alla Chem, LLC., Carson City NV 89701 (US)
(72) Inventor: ALLA CHEM, LLC, Carson City, NV 89701 (US); IVASHCHENKO, Alexander Vasilievich, Encinitas, CA 92024 (US); KYSIL, Volodymyr Mikhailovich, Kiev 021140 (UA); MITKIN, Oleg Dmitrievich, Khimki Moskovskaya obl. 141400 (RU); SAVCHUK Nikolay Filippovich, Rancho Santa Fe, CA 92067 (US)
(74) Representative: Henrion, Oliver
(86) International application number: PCT/RU2010/000216
(87) International publication number: WO 2010/134846

(57) **Abstract**

The invention relates to novel substituted 8-sulfonyl-2,3,4,5-tetrahydro-1*H*-γ-carbolines of the general formula **1** and their pharmaceutically acceptable salt - ligands exhibiting biological activity simultaneously towards alpha-adrenoceptors, dopamine receptors, histamine receptors, imidazoline receptors, sigma receptors, norepinefrine receptors, serotonin receptors, to active components, pharmaceutical compositions, comprising as an active component novel ligands, to novel medicaments intended for treatment of conditions and diseases of central nervous system. In the general formula 1 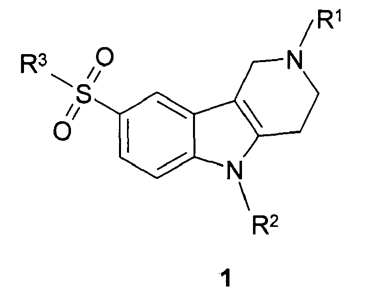 **R¹** represents a substituent selected from hydrogen, optionally substituted C₁-C₃ alkyl or C₁-C₄ alkyloxycarbonyl; **R²** represents a cyclic system substituent selected from hydrogen, optionally substituted C₁-C₃ alkyl, optionally substituted C₂-C₃ alkenyl or substituted sulfonyl; **R³** represents optionally substituted aryl or substituted amino group.

## Description

### Field of the invention

The invention relates to novel 2,3,4,5-tetrahydro-1*H-*γ-carbolines - ligands with wide range of biological activity, to active components, pharmaceutical compositions, comprising as an active component novel ligands, to novel medicaments for treatment of diseases and conditions of central nervous system (CNS) at humans and warm-blooded animals.

### Background of the invention

A great number of drugs and drug candidates for treatment of various conditions and diseases of CNS with wide range of receptor-specific activity are known. It makes possible to use such medicaments in wide range of therapeutic action. For example, (3aR,10cR)-2-methyl-1,2,3,3a,4,5,6,10c-octahydropyrrolo[3,4-c]carbazole **A** is α_{1A}-adrenoceptor, α_{1B}-adrenoceptor and α_{1D}-adrenoceptor antagonist and α₂-adrenoceptor agonist. This compound is a non-opioid analgetic [WO 1999065911].

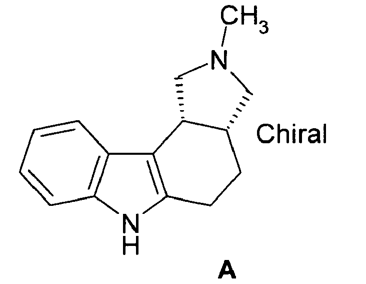

Central α₂-adrenoceptor antagonists stimulate noradrenaline release by blocking presynaptic α₂-receptors, which serve for negative control of this neurotransmitter release. By virtue of their ability to increase noradrenaline concentration, α₂-antagonists could be used for treatment or prophylaxis of depression. They are also potentially usefull for treatment of Alzheimer's disease and mnestic disorders, because it is known that α₂-antagonists promote acetylcholine release [Tellez, et al., J. Neurochem. 1997, 68, 778-785].

The range of Talipexole dihydrochloride **B** receptor activity, which is on the market since 1996 as a remedy for treatment of Parkinson's disease (PD), in addition to agonistic activity to dopamine D₂ receptors and dopamine autoreceptors, include agonistic activity to α₂-adrenoceptors [Boehringer Ingelheim, US 3804849].

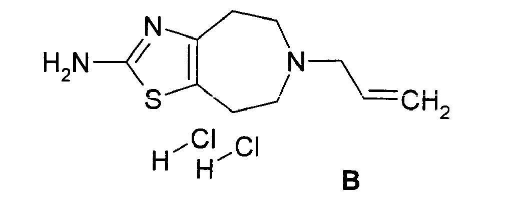

The range of Pardoprunox C receptor activity, which is in the III phase of clinical trial as a remedy for PD treatment, together with agonistic activity to 5-HT_{1A} receptors and partial agonistic activity to dopamine D₂ receptors, involve agonistic activity to α₁-adrenoceptors and antagonistic activity to α₂-adrenoceptors [Solvey, WO 2005107754].

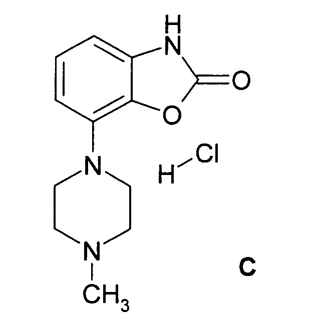

8-Chloro-11-piperazin-1-yl-5H-dibenzo[b,e][1,4]diazepine of Acadia Pharmaceuticals firm [WO 2006107948], the range of receptor-specific activity of which includes serotonin 5-HT_{2A} (inversive agonist), dopamine D₂ (partial agonist),dopamine D₃ (partial agonist) and muscarinic M₁ (agonist) activity, is in the II phase of clinical trial as a drug for treatment of PD.

γ-Carbolines of the general formula **D** exhibit high activity towards serotonin receptors, such as 5-HT₁ and 5-HT₂, α₂-adrenoceptors and dopamine receptors. These compounds are suitable for production of medicaments for depression, alarm conditions and psychosis therapy [Janssen Pharmaceutica Patent, US 6506768 B2, 2003]. 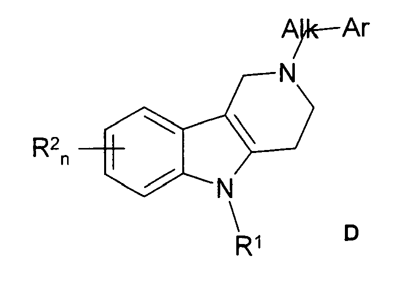 wherein: **R¹** represents hydrogen, optionally substituted C₁₋₆ alkyl, aryl; R² represents one or more substituents independently of each other selected from halogen, hydroxy, C₁₋₆ alkyl, C₁₋₆ alkoxy or nitro group; n= 0, 1, 2 or 3; Alk represents C₁₋₆ alkyldiyl; Ar represents optionally substituted azaheterocyclyl, optionally substituted phenyl or aryl.

The development of effective medicaments for anxiety disorders treatment (anxiolytics) has been intensively carried on for many years. It is known a great number of active ingredients, drugs and drug candidates for treatment of anxiety disorders, the mechanism of action of which is based upon their selective or wide range of receptor activity. As an example, it is possible to mention anxiolytics amongst α_{2A}-adrenoceptor antagonists [WO 2002066484], dopamine D₂ receptor antagonists [US 3816433, WO 2002043652, WO 2006096439, WO 2007058593], serotonin 5-HT_{1A} receptor agonists [WO 2004002487, WO 2005094827, WO 2000006163, EP 0280269, US 5183819, EP 0170213, WO 2001034136, WO 2005115396 , WO 2004069339 , WO 2007047978, WO 2005117886, WO 2004069339, WO 2007047978], serotonin 5-HT_{2A} receptor antagonists [US 4581171, WO 2006064754, EP 0066993, EP 0434021, WO 2001041766, WO 2001034136, EP 0374042, US 4737496, FR 2639942, WO 2007020337], serotonin 5-HT₆ receptor agonists [WO 2003053433, WO 2005014000, WO 2003101990], serotonin 5-HT₆ receptor antagonists [WO 2007108569, WO 2007054257, WO 2006091703, WO 2003035061, WO 2003072198, WO 2003011284, WO 2007098418].

For many years the development of effective medicaments for depression treatment (antidepressant) has been elaborated. It is known a great number of active ingredients, drugs and drug candidates for treatment of depression, the mechanism of action of which is based upon their selective or wide range of receptor activity (adrenoceptor, dopamine, serotonin and other). As example, Table 1 represents some known active components for treatment of depression.

**Table 1 Active components for depression treatment.**

| **Formula, name** | **Patent** | **Therapeutical group** | **Phase** | **Mechanism of action** |
|---|---|---|---|---|
| 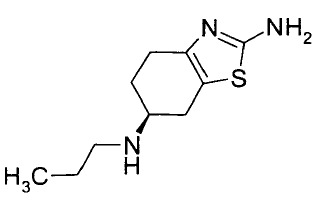 | Boehringer Ingelheim, WO 2007075095 | Antidepressant and other | On the market since 1997 | Dopamine D₃ agonist |
| 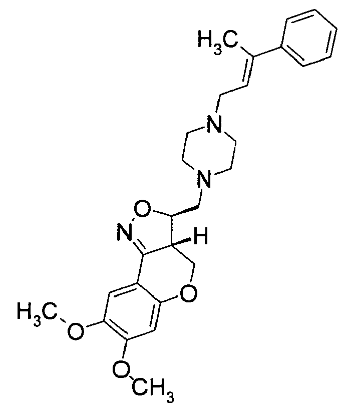 | Johnson & Johnson, WO 2002066484 | Antidepressant antagonist, | Preclinica 1 | α_{2A(2D)}-adrenoceptor antagonist, α_{2C}-adrenoceptor antagonist, serotonin transporter (SERT) inhibitor |
| 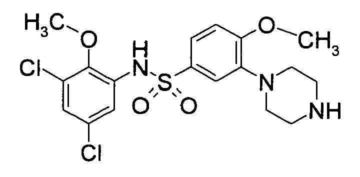 | GlaxoSmith Kline, WO 2003072198 | Antidepressant and other | Preclinica 1 | 5-HT₆ antagonist |
| 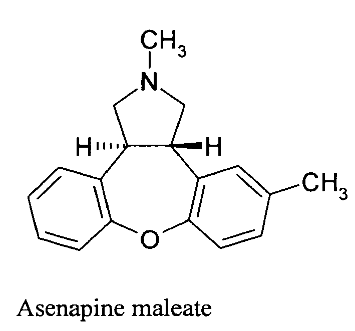 | Organon, Pfizer WO 2006040314 | Bipolar disorder and other | Phase III | Dopamine D₁ antagonist, dopamine D₂ antagonist, 5-HT₂ antagonist |
| 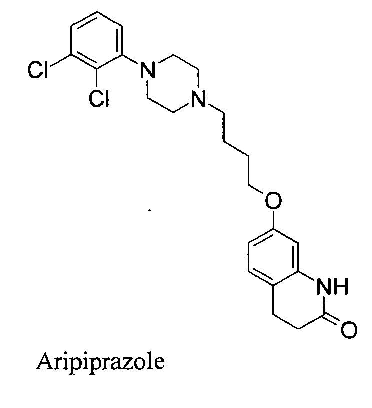 | Otsuka and others WO 2004060374 | Antidepressant and other. | On the market since 2002 | Dopamine D₂ partial agonist, 5-HT_{1A} partial agonist, 5-HT₂ antagonist |
| 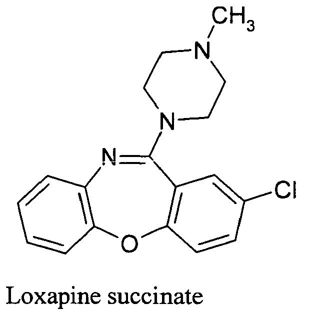 | White Pharmaceuti ca WO 2005023243 | Antidepressant and other. | On the market since 1975 | Dopamine D₂ antagonist, dopamine D₁ ligand, dopamine D₃ ligand, dopamine D₄ ligand, 5-HT₂ antagonist |

For many years the development of effective medicaments for treatment of psychotic illnesses (antipsychotics) has been carried out. It is known a great number of active ingredients, drugs and drug candidates for treatment of psychotic diseases, the mechanism of action of which is based upon their selective or wide range of receptor activity. As an example, Table 2 represents some known active components for psychotic illnesses treatment.

**Table 2 Active ingredients for psychotic diseases treatment.**

| **Formula, name** | **Patent** | **Therapeutical group** | **Phase** | **Mechanism of action** |
|---|---|---|---|---|
| 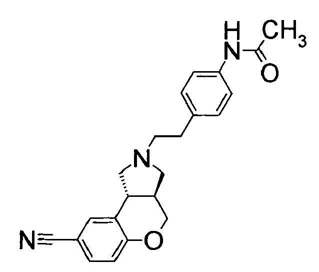 | Servier (Originator) EP 0887350 | Antipsychotic | Phase I | Dopamine D₃ antagonist |
| 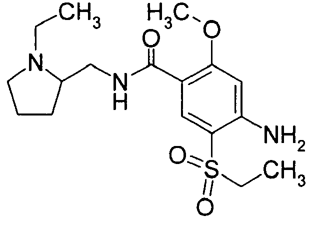 | GlaxoSmith Kline, Snofi Aventis, WO 2006048560 | Antipsychotic | On the market since 1986 | Dopamine D₂ antagonist, dopamine D₃ antagonist |
| 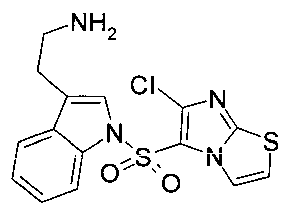 | Wyeth Pharmaceuticals WO 2003053433 | Antipsychotic and other | Phase I | serotonin 5-HT₆ antagonist |
| 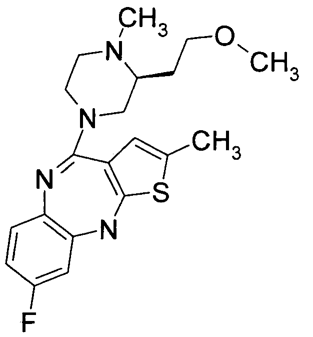 | Lilly WO 2004014895 | Antipsychotic | | Dopamine D₂ antagonist, serotonin 5-HT_{2A} antagonist, 5-HT₆ antagonist |
| 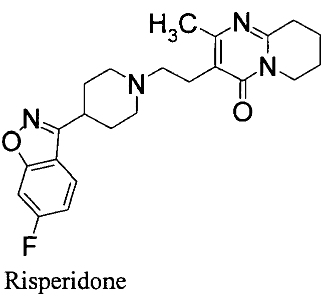 | Janssen WO 2000023057 | Antipsychotic and other | On the market since 1993 | Dopamine D₂ antagonist, 5-HT_{2A} antagonist |
| 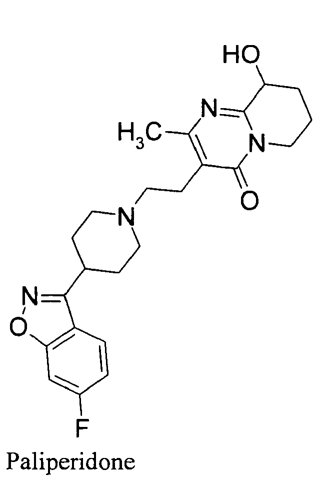 | Janssen WO 2007044234 | Antipsychotic | On the market since 1997 | Dopamine D₂ antagonist, 5-HT₂ antagonist |
| 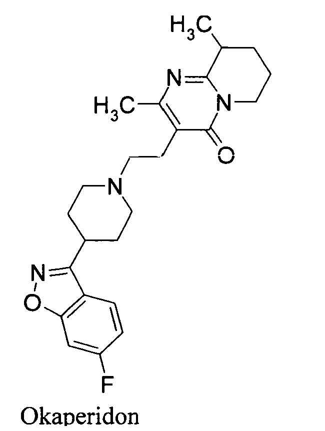 | Janssen Pharmaceuti c EP 0196132, EP 0453042 | Antipsychotic | Phase II | Dopamine D₂ antagonist, 5-HT_{2A} antagonist |
| 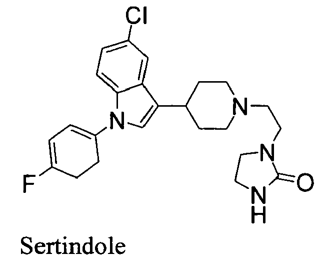 | Shionogi, WO 1997039752 | Antipsychotic | On the market since 2006 | Dopamine D₂ antagonist, 5-HT_{2A} antagonist |
| 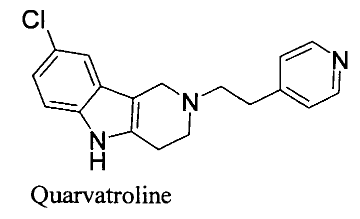 | American Home Products Corporation US 4636563 | Antipsychotic | Phase 1 | Dopamine D₂ antagonist, 5-HT_{2A} antagonist |
| 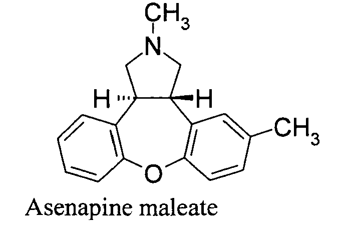 | Organon, Pfizer, WO 2006040314 | Antipsychotic and other | Phase III | Dopamine D₁ antagonist, dopamine D₂ antagonist, 5-HT₂ antagonist |
| 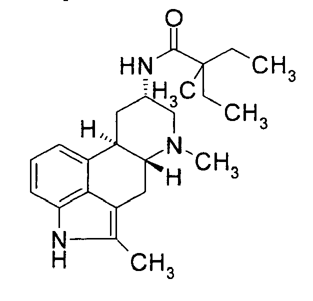 | Ho 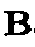 ap 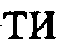 c GB 2206115 | Antipsychotic and other ic and other | Phase II | Dopamine D₂ partial agonist, dopamine D₁ antagonist, 5-HT_{1A} partial agonist |
| 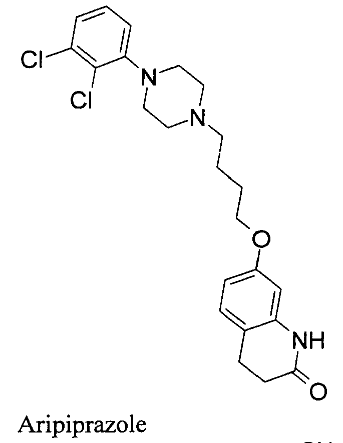 | Otsuka and others WO 2004060374 | Autism. Antipsychotic and other | On the market since 2002 | Dopamine D₂ partial agonist, 5-HT_{1A}partial agonist, 5-HT₂ antagonist |
| 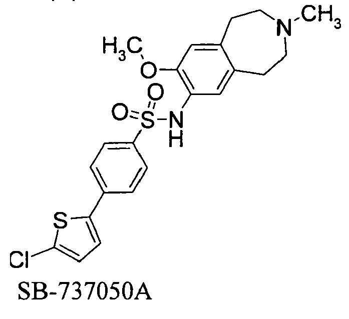 | Glaxo group WO 2003068752 | Antipsychotic | Phase II | Dopamine D₂ antagonist, dopamine D₃ antagonist, 5-HT₆ antagonist, 5-HT_{2A} antagonist, 5-HT₂c antagonist |
| 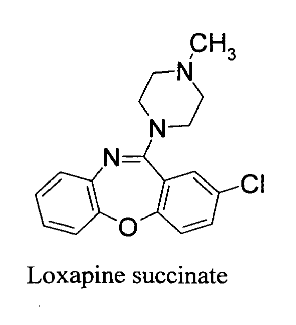 | White Pharmaceutica WO 2005023243 | Antipsychotic and other | On the market since 1975 | Dopamine D₂ antagonist, dopamine D₁ ligand, dopamine D₃ ligand, dopamine D₄ ligand, 5-HT₂ antagonist |

The development of novel effective remidies for treatment of neurodegenerative diseases (ND) and cognitive disorders (CD) has been carried on extensively for many years. There are known the whole number of medicaments and drug candidates for treatment of ND and CD, mechanism of action of which is associated with their ability to interact with one or more receptors, including: α-adrenoceptors, dopamine receptors, serotonin receptors and others. As an example of such active components dopamine D₁ receptor agonists for Parkinson's disease treatment [Novartic patent DE 3402392; Darpharma and others patent WO 2006012640; Smithkline Beecham patent, WO 1996039136; Nastech Pharmaceutical company patent WO 2002024202; Maruka patent and others, JP 1988033377] could be given. One of the most promising approach to the treatment of Alzheimer's and other neurodegenerative diseases is based upon the usage of effective medicaments, active towards serotonin 5-HT₆ receptors [Holenz J., Pauwels P.J., Diaz J.L., Merce R., Codony X., Buschmann H. Medicinal chemistry strategies to 5-HT6 receptor ligands as potential cognitive enhancers and antiobesity agents. Drug Disc. Today. 2006; 11:283-299]. At mammals these receptors are localized exclusively in central nervous system (CNS), and mainly in the parts of brain responsible for training and memory [Ge'rard C., Martres M.-P., Lefe'vre K., Miquel M.-C., Verge' D., Lanfumey L., Doucet E., Hamon M., El Mestikawy S. Immuno-localisation of serotonin 5-HT6 receptor-like material in the rat central nervous system. Brain Research. 1997; 746:207-219]. Besides, it was shown that 5-HT₆ receptors were the whole number of neuromediator system modulators including cholinergic, noradrenergic, glutamatergic and dopaminergic [Dawson L.A., Nguyen H.Q., Li P. The 5-HT(6) receptor antagonist SB-271046 selectively enhances excitatory neurotransmission in the rat frontal cortex and hippocampus. Neuropsychopharmacology. 2001; 25:662-668]. Taking into account the fundamental role of these systems in normal cognitive processes and their dysfunction at neurodegeneration, exclusive role of 5-HT₆ receptors in normal and "pathological" memory formation becomes obvious. In a great number of nowadays publications it was shown that blocking of 5-HT₆ receptors leads to a considerable enhancement of memory consolidation in various animal's models of training-memorizing-replicating [Foley A.G., Murphy K.J., Hirst W.D., Gallagher H.C., Hagan J.J., Upton N., Walsh F.S., Regan C.M. The 5-HT(6) receptor antagonist SB-271046 reverses scopolamine-disrupted consolidation of a passive avoidance task and ameliorates spatial task deficits in aged rats. Neuropsychopharmacology. 2004; 29:93-100. Riemer C., Borroni E., Levet-Trafit B., Martin J.R., Poli S., Porter R.H., Bos M. Influence of the 5-HT6 receptor on acetylcholine release in the cortex: pharmacological characterization of 4-(2-bromo-6-pyrrolidin-l-ylpyridine-4-sulfonyl)phenylamine, a potent and selective 5-HT6 receptor antagonist. J. Med. Chem. 2003; 46:1273-1276. King M.V., Woolley M.L., Topham LA., Sleight A.J., Marsden C.A., Fone K.C. 5-HT6 receptor antagonists reverse delay-dependent deficits in novel object discrimination by enhancing consolidation an effect sensitive to NMDA receptor antagonism. Neuropharmacology 2004; 47:195-204]. It was also shown that considerable enhancement of cognitive functions in aged rats in Morrison's water maze experiment took place under the action of 5-HT₆ receptor antagonists [Foley A.G., Murphy K.J., Hirst W.D., Gallagher H.C., Hagan J.J., Upton N., Walsh F.S., Regan C.M. The 5-HT(6) receptor antagonist SB-271046 reverses scopolamine-disrupted consolidation of a passive avoidance task and ameliorates spatial task deficits in aged rats. Neuropsychopharmacology. 2004; 29:93-100]. Recently more thorough understanding of 5-HT₆ receptor function in cognitive processes and more accurate conceptions concerning possible pharmacophoric properties of their antagonists were achieved. [Holenz J., Pauwels P.J., Diaz J.L., Merce R., Codony X., Buschmann H. Medicinal chemistry strategies to 5-HT6 receptor ligands as potential cognitive enhancers and antiobesity agents. Drug Disc. Today. 2006; 11:283-299]. This resulted in preparation of highly affine selective ligands ("molecular tools"), and afterwards clinical candidates. At present a number of 5-HT₆ receptor antagonists are at various phases of clinical trials as potential ingredients for treatment of AD, Huntington's disease, schizophrenia (antipsychotic) and other neurodegenerative and cognitive diseases (Table 1) [http://integrity.prous.com].

### Disclosure of the invention

The authors of the invention disclosed novel ligands with broad range of biological activity including simultaneously α-adrenoceptors, dopamine receptors, histamine, imidazoline, sigma receptors, norepinephrine receptors, and serotonin receptors. The authors displayed the possibility of their use as an active component for pharmaceutical compositions and medicaments for treatment and prophylaxis of various pathological conditions and diseases of CNS.

In the context of the invention, the terms are generally defined as follows:
**"Agonists"** mean ligands which being bound to receptors of definite type actively promote transferring their specific signal and by that cause the biological response of the cell.
**"Azaheterocycle"** means aromatic or nonaromatic mono- or polycyclic system with at least one nitrogen atom. Azaheterocycles may have one or more "cyclic system substituents".
**"Active component"** (drug-substance) means physiologically active compound of synthetic or other (biotechnological, vegetable, animal, microbe and so on) origins exhibiting pharmacological activity which is an active ingredient of pharmaceutical composition employing in production and preparation of medicaments.
**"Alkenyl"** means aliphatic straight or branched hydrocarbon chain, comprising 2-7 carbon atoms and including carbon-carbon double bond. Branched means that the straight alkenyl chain contains one or more lower alkyl groups, such as methyl, ethyl or propyl. Alkyl group may have one or more substituents, for example, halogen, alkenyloxy, cycloalkyl, cyano, hydroxy, alkoxy, carboxy, alkynyloxy, aralkoxy, aryloxy, aryloxycarbonyl, alkylthio, heteroaralkyloxy, heterocyclyl, heterocyclylalkyloxy, alkoxycarbonyl, aralkoxycarbonyl, heteroaralkyloxycarbonyl or G¹G²N-, G¹G²NC(=O)-, G¹G²NSO₂-, wherein G¹ and G² independently of each other represent hydrogen, alkyl, aryl, aralkyl, heteroaralkyl, heterocyclyl or heteroaryl, or G¹ and G² together with the nitrogen atom they are attached to form through G¹ and G² 4 - 7 membered heterocyclyl or heterocyclenyl. The preferred alkyl groups are methyl, trifluoromethyl, cyclopropylmethyl, cyclopentylmethyl, ethyl, *n*-propyl, *iso*-propyl, *n-*butyl, *tert*.-butyl, *n*-pentyl, 3-pentyl, methoxyethyl, carboxymethyl, methoxycarbonylmethyl, benzyloxycarbonylmethyl and pyridylmethyloxycarbonylmethyl. The preferred alkenyl groups are ethenyl, propenyl, *n*-butenyl, *iso*-butenyl, 3-methylbut-2-enyl, pentenyl and cyclohexylbutenyl.
**"Alkyl"** means aliphatic hydrocarbon straight or branched chain with 1-12 carbon atoms. Branched means alkyl chain with one or more "lower C₁-C₄ alkyl" substituents. Alkyl group may have one or more substituents of the same or different structure ("alkyl substituent") including halogen, alkenyloxy, cycloalkyl, aryl, heteroaryl, heterocyclyl, aroyl, cyano, hydroxy, alkoxy, carboxy, alkynyloxy, aralkoxy, aryloxy, aryloxycarbonyl, alkylthio, heteroarylthio, aralkylthio, arylsulfonyl, alkylsulfonyl, heteroaralkyloxy, annelated heteroarylcycloalkenyl, annelated heteroarylcycloalkyl, annelated heteroarylheterocyclenyl, alkylsulfonylheteroaralkyloxy, annelated heteroarylheterocyclyl, annelated arylcycloalkenyl, annelated arylcycloalkyl, annelated arylheterocyclenyl, annelated arylheterocyclyl, alkoxycarbonyl, aralkoxycarbonyl, heteroaralkyloxycarbonyl or G¹G²N-, G¹G²NC(=O)-, G¹G²NC(=S)-, G¹G²NSO₂-, where G¹ and G² independently of each other represent hydrogen, alkyl, aryl, aralkyl, heteroaralkyl, heterocyclyl or heteroaryl, or G¹ and G² together with the N-atom, they are attached to, form through G¹ and G² 4-7-membered heterocyclyl or heterocyclenyl. The preferred alkyl groups are methyl, trifluoromethyl, cyclopropylmethyl, cyclopentylmethyl, ethyl, *n*-propyl, *iso*-propyl, *n*-butyl, *tert.*-butyl, *n*-pentyl, 3-pentyl, methoxyethyl, carboxymethyl, methoxycarbonylmethyl, ethoxycarbonylmethyl, benzyloxycarbonylmethyl and pyridylmethyloxycarbonylmethyl. The preferred "alkyl substituents" are cycloalkyl, aryl, heteroaryl, heterocyclyl, hydroxy, alkoxy, alkoxycarbonyl, aralkoxy, aryloxy, alkylthio, heteroarylthio, aralkylthio, alkylsulfonyl, arylsulfonyl, alkoxycarbonyl, aralkoxycarbonyl, heteroaralkyloxycarbonyl or G¹G²N-, G¹G²NC(=O)-, annelated arylheterocyclenyl, annelated arylheterocyclyl.
**"Alkoxy"** means alkyl-O-group, wherein alkyl is defined in this section. The preferred alkoxy groups are methoxy, ethoxy, *n*-propoxy, *iso*-propoxy and *n*-butoxy.
**"Alkyloxycarbonyl"** means alkyl-O-C(=O)- group, wherein alkyl is defined in this section. The preferred alkoxycarbonyl groups are methoxycarbonyl, ethoxycarbonyl and *tert.-*butyloxycarbonyl.
**"Amino group"** means G¹G²N- group, substituted or unsubstituted with "amino group substituent" G¹ and G², the meanings of which are defined in this section, for example, amino (H₂N-), methylamino, diethylamino, pyrrolidino, morpholino, benzylamino or phenethylamino. **"Anxiolytic"** (tranquilizer) means a medicament intended for anxious disorders treatment.
**"Antagonists"** mean ligands which being bound to definite receptors do not cause active cellular responses. Antagonists prevent linkage between agonists and receptors and by that blocking specific receptor signal transmission.
**"Antidepressant"** means medicament intended for depression treatment.
**"Antipsychotic"** means remedy intended for psychotic diseases treatment.
**"Aryl"** means aromatic mono- or polycyclic system with 6 - 14 carbon atoms, predominantly 6-10 carbon atoms. Aryl may have one or more "cyclic system substituents" of the same or different structure. Phenyl, substituted phenyl, naphthyl, or substituted naphthyl are the representatives of aryl groups. Aryl could be annelated with nonaromatic cyclic system or heterocycle.
**"Arulsulfonyl"** means aryl-SO₂- group, wherein meaning of aryl is defined in this section.
**"Addictive substances"** mean medicaments, narcotics, psychoactive and physiologically active compounds, capable to cause addiction and dependence, such as alcohol, nicotine, amphetamines or sympathomimetics similar in their action; caffeine and its analogs, cannabinoids, cocaine and its analogs, hallucinogens, inhaled compounds, opiates, phencyclidine and its analogs, muscle pills, hypnotic and sedative drugs, and also any other natural, semisynthetic, synthetic or biotechnological compounds or mixtures of them capable to cause addiction and dependence.
**"1,2-Vinyl radical**" means -CH=CH- group, which comprises one or more "alkyl substituents of the same or different structure, the meanings of which are defined in this section. **"Halogen"** means fluorine, chlorine, bromine and iodine. Preference is given to fluorine, chlorine and bromine.
**"Heteroaryl"** means an aromatic mono- or polycyclic system with 5 - 14 carbon atoms, preferably from 5 to 10, wherein one or more carbon atoms are substituted by one or more heteroatoms, such as N, S or O. Prefix "aza", "oxa" or "thia" before "heteroaryl" means that N, O or S atoms are introduced in the appropriate cyclic fragment. N-Atom of heteroaryl cycle could be oxidized to N-oxide. Heteroaryl may have one or more "cyclic system sustituents" of the same or different structure. Pyrrolyl, furanyl, thienyl, pyridyl, pyrazinyl, pyrimidinyl, isoxazolyl, isothiazolyl, tetrazolyl, oxazolyl, thiazolyl, pyrazolyl, furazanyl, triazolyl, 1,2,4-thiadiazolyl, pyridazinyl, quinoxalinyl, phthalazinyl, imidazo[1,2-a]pyridinyl, imidazo[2,1-b]thiazolyl, benzofurazanyl, indolyl, azaindolyl, benzoimidazolyl, benzothiazenyl, quinolinyl, imidazolyl, thienopyridyl, quinazolinyl, thienopyrimidinyl, pyrrolopyridinyl, imidazopyridinyl, isoquinolinyl, benzoazaindolyl, 1,2,4-triazinyl, thienopyrrolyl, furopyrrolyl and others are the representatives of heteroaryl radicals.
**"Heterocycle"** means an aromatic or nonaromatic mono- or polycyclic system comprising at least one heteroatom. The preferred heteroatoms are nitrogen, oxygen and sulfur. Azaheterocycle may have one or more "cyclic system substituents".
**"Heterocyclyl"** means a radical derived from heterocycle.
**"Hydrate"** means stoichiometric or nonstoichiometric compositions of the compounds or their salts with water.
**"Depression"** means big depression; incidental, chronic and recurring form of big depression; dysthymic disorder (dysthymia); cyclotymia; affective disorder; syndrome of seasonal affective disorder; bipolar disorder, including bipolar disorders of I and II types; and other depressive disorders and conditions. Depression also means the depressions accompanying AD, vascular dementia; disorder of mood induced by alcohol and substances; schizoaffective disorder of depressive type; disorder of adaptation. Besides, depression includes depression of oncological patients; depression at Parkinson's disease; depressions after myocardial infarction; depressions of fruitless women; pediatric depression; postnatal depression; the depressions accompanying somatic, neuralgic and other diseases.
**"Substituent"** means a chemical radical attached to a scaffold (fragment), for example, "alkyl substituent", "amino group substituent", "carbamoyl substituent", and "cyclic system substituent", the meanings thereof are defined in this section.
**"Alkyl group substituent"** means a substituent attached to alkyl or alkenyl group, the meanings of which are defined in this section. Alkyl group substituent is selected from hydrogen, alkyl, halogen, alkenyloxy, cycloalkyl, aryl, heteroaryl, heterocyclyl, aroyl, cyano, hydroxy, alkoxy, carboxy, alkynyloxy, aralkoxy, aryloxy, aryloxycarbonyl, alkylthio, heteroarylthio, aralkylthio, arylsulfonyl, alkylsulfonyl, heteroaralkyloxy, annelated heteroarylcycloalkenyl, annelated heteroarylcycloalkyl, annelated heteroarylheterocyclenyl, annelated heteroarylheterocyclyl, annelated arylcycloalkenyl, annelated arylcycloalkyl, annelated arylheterocyclenyl, annelated arylheterocyclyl, alkoxycarbonyl, aralkoxycarbonyl, heteroaralkyloxycarbonyl or G¹G²N-, G¹G²NC(=O)-, G¹G²NSO₂-, where G¹ and G² independently of each other represent hydrogen, alkyl, aryl, aralkyl, heteroaralkyl, heterocyclyl or heteroaryl, or G¹ and G² together with N-atom they are attached to via G¹ and G² form 4-7-membered heterocyclyl or heterocyclenyl. The preferred alkyl groups are methyl, trifluoromethyl, cyclopropylmethyl, cyclopentylmethyl, ethyl, *n*-propyl, *iso*-propyl, *n*-butyl, *tert*.-butyl, *n*-pentyl, 3-pentyl, methoxyethyl, carboxymethyl, methoxycarbonylmethyl, ethoxycarbonylmethyl, benzyloxycarbonylmethyl, and pyridylmethyloxycarbonylmethyl. The preferred "alkyl group substituents" are cycloalkyl, aryl, heteroaryl, heterocyclyl, hydroxy, alkoxy, alkoxycarbonyl, aralkoxy, aryloxy, alkylthio, heteroarylthio, aralkylthio, alkylsulfonyl, arylsulfonyl, alkoxycarbonyl, aralkoxycarbonyl, heteroaralkyloxycarbonyl or G¹G²N-, G¹G¹NC(=O)-, annelated arylheterocyclenyl, annelated arylheterocyclyl.
**"Amino group substituent" means** a substituent attached to amino group. Amino group substituent represents hydrogen, alkyl, cycloalkyl, aryl, heteroaryl, heterocyclyl, acyl, aroyl, alkylsulfonyl, arylsulfonyl, heteroarylsulfonyl, alkylaminocarbonyl, arylaminocarbonyl, heteroarylaminocarbonyl, heterocyclylaminocarbonyl, alkylaminothiocarbonyl, arylaminothiocarbonyl, heteroarylaminothiocarbonyl, heterocyclylaminothiocarbonyl, annelated heteroarylcycloalkenyl, annelated heteroarylcycloalkyl, annelated heteroarylheterocyclenyl, annelated heteroarylheterocyclyl, annelated arylcycloalkenyl, annelated arylcycloalkyl, annelated arylheterocyclenyl, annelated arylheterocyclyl, alkoxycarbonylalkyl, aralkoxycarbonylalkyl, heteroaralkyloxycarbonylalkyl.
**"Cyclic system substituent"** means a substituent attached to an aromatic or nonaromatic cyclic system, among them hydrogen, alkyl, alkenyl, alkynyl, aryl, heteroaryl, aralkyl, heteroaralkyl, hydroxy, hydroxyalkyl, alkoxy, aryloxy, acyl, aroyl, halogen, nitro, cyano, carboxy, alkoxycarbonyl, aryloxycarbonyl, aralkoxycarbonyl, alkylsulfonyl, arylsulfonyl, heteroarylsulfonyl, alkylsulfinyl, arylsulfinyl, heteroarylsulfinyl, alkylthio, arylthio, heteroarylthio, aralkylthio, heteroaralkylthio, cycloalkyl, cycloalkenyl, heterocyclyl, heterocyclenyl, amidino, G¹G²N-, G¹G²N-alkyl-, G¹G²NC(=O)- or G¹G²NSO₂-, where G¹ and G² independently of each other represent hydrogen, optionally substituted alkyl, optionally substituted aryl, optionally substituted aralkyl, optionally substituted heteroaralkyl, or G¹G²N-substituent in which one of G¹ and G² could be acyl or aroyl, and the meaning of the other of G¹ and G² is defined above, or "cyclic system substituent" is G¹G²NC(=O)- or G¹G²NSO₂-, in which G¹ and G² together with the nitrogen atom they are attached to form through G¹ and G² 4-7-membered heterocyclyl or heterocyclenyl. The preferred "cyclic system substituents" are alkoxycarbonyl, alkoxy, halogen, aryl, aralkoxy, alkyl, hydroxy, aryloxy, nitro, cyano, alkylsulfonyl, heteroaryl or G¹G²N-. If cyclic system is saturated or partly saturated, "cyclic system substituent" may get the meaning methylene (CH₂=), oxo (O=) or thioxo (S=).
**"Cognitive disorders" or disorders of cognitive functions"** mean disorder (weakening) of mental abilities including attentiveness, memory, mentality, cognition, education, verbal, mental, executive and creative abilities, time and space orientation; in particular, cognitive disorders associated with Alzheimer's disease, Parkinson's and Huntington's diseases, senile dementia; age-associated memory impairment, AAMI; dysmetabolic encephalopathy; psychogenous memory impairment; amnesia; amnesic disturbances; transit global amnesia; dissociative amnesia; vascular dementia; light or mild cognitive impairment, MCI; attention deficit hyperactivity disorder (AD/HD); cognitive impairments, accompanying psychotic diseases, epilepsy, delirium, autism, psychosis, Down's syndrome, bipolar disorders and depression; AIDS-associated dementia; dementias at hypothyroidism; dementia connected with alcohol, substances causing dependability and neurotoxins; dementia accompanying neurodegenerative diseases, for example, cerebellar degeneracy and amyotrophic lateral sclerosis; cognitive disturbances connected with cerebral crisis, infectious and oncological brain diseases as well as traumatic brain injury; cognitive functions damages associated with autoimmune and endocrine diseases, and others.
**"Medicament" -** is a compound (or a mixture of compounds in the form of pharmaceutical composition) in the form of tablets, capsules, injections, ointments and other ready forms intended for restoration, improvement or modification of physiological functions at humans and animals, and for treatment and prophylaxis of diseases, diagnostics, anesthesia, contraception, cosmetology and others.
**"Ligand"** (from Latin *ligo*) represents a chemical compound (small molecule, peptide, protein, inorganic ion, and so on) capable to interact with receptors which convert this interaction into specific signal.
**"Neurodegenerative diseases"** mean specific conditions and diseases, accompanied by damage and primary destruction of nervous cell populations in certain areas of central nervous system. Neurodegenerative diseases include but are not limited by: Alzheimer's disease, Parkinson's and Huntington's diseases (chorea); multiocular sclerosis; cerebellar degeneracy; amyotrophic lateral sclerosis; dementias with Lewy bodies; spinal muscular atrophy; peripherical neuropathy; spongy encephalitis (Creutzfeld-Jakob Disease); AIDS dementia; multi-infract dementia; frontotemporal dementias; leukoencephalopathy (spongy degeneration of white matter); chronic neurodegenerative diseases; insult; ischemic, reperfusion and hypoxic brain damage; epilepsy; cerebral ischemia; glaucoma; traumatic brain injury; Down's syndrome; encephalomyelitis; meningitis; encephalitis; neuroblastoma; schizophrenia; depression. Moreover, neurodegenerative diseases include pathological states and disorders associated with hypoxia, substance abuse, causing dependability, under neurotoxins influence; infectious and ontological brain diseases as well as neuronal damages associated with autoimmune and endocrine diseases and others.
**"Optionally substituted radical"** means a radical without substituent or a radical with one or more substituents.
**"Lowre alkyl"** means straight or branched alkyl with 1-4 carbon atoms.
**"Nootrops"** or **"Nootropics"** (*neurometabolic stimulates*) are medicaments taken for cognition enhancement.
**"Psychic disorders, (psychic diseases)"** are diseases or diseased states associated with mental disturbance and/or mentality frustration. "Psychic disorders" include affective disorders (bipolar affective disorders, big depression, hypomania, minor depression, maniacal syndrome, Cotard's syndrome, cyclothymia, schizoaffective disorders and so on), intellectual-mnestic disorders; manias (hypomania, graphomania, kleptomania, compulsive shopping, mania of persecution, pornographomania, erotomania and so on); disorder of multiple personality, amentia, alcoholomania, deliration, delirium syndrome, hallucinosis, hallucinations, lucinatory effects, homicidomania, delirium; illusion, querulous paranoiaclinical lycanthropy, macropsia, antagonistic delusion, micropsia, narcomania; anorexia nervosa, oneiroid syndrome, paranoid, paranoia, paraphrenia, pseudohallucinations, psychosis, Cotard's syndrome, schizoaffective disorder, schizotypical disorder, schizophrenia, schizoaffective psychosis disorder, schizophrenomorphic disorder, Shrebera's syndrome, Daniel Paul's syndrome), phobias (agarophobia, arachnephobia, autophobia, verminophobia, hydrosophobia, hydrophobia, demophobia, zoophobia, carcinophobia, claustrophobia, climacophobia, xenophobia, misophobia, radiophobia, photophobia, skoliephobia, scotophobia, social phobia, tetraphobia, triskaidekaphobia, erotophobia); alcoholic psychosis, alcoholic palimpsest, allotriophagy, aphasia, graphomania, dissociative fugue state, dissociative disorders; dysphorias, internet-dependences, hypochondria, hysteria, kopophobia, delirium of persecution, melancholy, misanthropy, obsession, panic attacks, Asperger's syndrome, Capgras' syndrome, Munchausen's syndrome, Retta's syndrome, Fregoly's syndrome, syndrome of attention and hyperactivity deficit, obsessive-compulsive disorder, syndrome of chronic narcotization consequences, syndrome of psychic automatism, syndrome of infantile autism, madness, taphophilia, anxiety conditions, Hikikomory's syndrome, erotographomania and so on.
**"Psychotic diseases"** are all types of schizophrenia; schizoaffective psychosis; schizotypical disorders; schizoaffective disorders, including bipolar and depressive types; delirious disorders including reference delusion, delusion of persecution, megalomania, delusion of jealousy, erotomania, and also hypochondriacal, somatic, mixed and not differentiated delirium; short-time psychotic disorders; induced psychotic frustration; induced by substances psychotic frustration; and other psychotic disorders.
**"Substance-related disorder"** means substance use disorders, such as, substance dependence for example, drug substances, alcohol, narcotics (addiction), substance craving and substance abuse; and disorders induced by chemical compounds, such as, substance intoxication, abstinency or withdrawal symptoms, substance-induced delirium, substance-induced persisting dementia, substance-induced persisting amnesic disorder, substance-induced psychotic disorder, substance-induced mood disorder, substance-induced anxiety disorder, substance-induced sexual dysfunction, substance-induced sleep disorder, substance-induced persisting perception disorder, flashbacks.
**"Receptors"** (from latin *recipere*) represent biological macromolecules located either on cytoplasmic cell membrane or intracellular, capable specifically interact with restricted number of physiologically active compounds (ligandes) and transform the signal about this interaction into definite cellular response.
**"Anxiety disorders"** means generalized (inconcrete) anxiety; acute uncontrolled anxiety; panic disorder; phobia, for example, agoraphobia (acute fear of crowded place) or social (acute fear of humiliation at presence of other people) or any other phobia (acute fear of particular subjects, animals or situations , in the form of phobia of height, of medical procedures, lifts, open space etc.); an obsessional condition (obsessive-compulsive disorder); posttraumatic stress disorder and acute stress disorder. Besides, anxiety disorders include anxiety conditions induced by alcohol or substances; anxiety under adaptation; as well as mixed forms of anxiety disorders and depression.
**"Schizophrenia"** means all known types, forms and variants of the disease, including: simple, hebephrenic, paranoid, hypertoxic (pyretic), catatonic, schizoaffective, residual or not differentiated schizophrenia and/or the forms of schizophrenia defined in classification of American Psychiatric Association (*American Psychiatric Association; in: Diagnostic and Statistical Manual of Mental Disorders,* IV Edition, Washington D.C. 2000) or in International classification (*International Statistical Classification of Diseases and Related Health Problems*) or any other known forms.
**"Pharmaceutical composition"** means a composition comprising active component and at least one of the components selected from a group consisting of pharmaceutically acceptable and pharmacologicaly compatible fillers, solvents, diluents, auxiliary, distributing and sensing agents, delivery agents, such as preservatives, stabilizers, disintegrators, moisteners, emulsifier, suspending agents, thickeners, sweeteners, flavouring agents, aromatizing agents, antibacterial agents, fungicides, lubricants, and prolonged delivery controllers, the choice and suitable proportions of which depend on the nature and the way of administration and dosage. Examples of suitable suspending agents are ethoxylated isostearyl alcohol, polyoxyethene, sorbitol and sorbitol ether, microcrystalline cellulose, aluminum metahydroxide, bentonite, agar-agar and tragacant and their mixtures as well. Protection against the action of microorganisms can be provided by various antibacterial and antifungal agents, such as, for example, parabens, chlorobutanole, sorbic acid, and similar compounds. A composition may also contain isotonic agents, such as, for example, sugar, sodium chloride, and similar compounds. A prolonged effect of the composition may be achieved by agents slowing down absorption of the active ingredient, for example, aluminum monostearate and gelatine. Examples of suitable carriers, solvents, diluents and delivery agents include water, ethanol, polyalcohols and their mixtures, natural oils (such as olive oil) and organic esters (such as ethyl oleate) for injections. Examples of fillers are lactose, milk-sugar, sodium citrate, calcium carbonate, calcium phosphate and the like. Examples of disintegrators and distributors are starch, alginic acid and its salts, and silicates. Examples of suitable lubricants are magnesium stearate, sodium lauryl sulfate, talc and high molecular weight polyethylene glycol. A pharmaceutical composition for peroral, sublingval, transdermal, intramuscular, intravenous, subcutaneous, local or rectal administration of the active ingredient, alone or in combination with another active compound may be administered to humans and animals in a standard administration form, or in a mixture with traditional pharmaceutical carriers. Suitable standard administration forms include peroral forms such as tablets, gelatin capsules, pills, powders, granules, chewing-gums and peroral solutions or suspensions, for example, therapeutic kit; sublingval and transbuccal administration forms; aerosols; implants; local, transdermal, subcutaneous, intramuscular, intravenous, intranasal or intraocular forms and rectal administration forms.
**"Pharmaceutically acceptable salt"** means relatively nontoxic both organic and inorganic salts of acids and bases disclosed in this invention. The salts could be prepared in situ in the processes of synthesis, isolation or purification of compounds or they could be prepared specially. In particular, bases salts could be prepared starting from purified base of the disclosed compound and suitable organic or mineral acid. Examples of salts prepared in this manner include hydrochlorides, hydrobromides, sulfates, bisulfates, phosphates, nitrates, acetates, oxalates, valeriates, oleates, palmitates, stearates, laurates, borates, benzoates, lactates, p-toluenesulfonates, citrates, maleates, fumarates, succinates, tartrates, methane sulphonates, malonates, salicylates, propionates, ethane sulphonates, benzene sulfonates, sulfamates and the like (Detailed description of properties of such salts is given in: Berge S.M., et al., "Pharmaceutical Salts" J.Pharm.Sci., 1977, 66: 1-19). Salts of the disclosed acids may be also prepared by the reaction of purified acids specifically with a suitable base; moreover, metal salts and amine salts may be synthesized too. Metal salts are salts of sodium, potassium, calcium, barium, magnesium, lithium and aluminum, sodium and potassium salts being preferred. Suitable inorganic bases from which metal salts can be prepared are sodium hydroxide, carbonate, bicarbonate and hydride; potassium hydroxide, carbonate and bicarbonate, lithium hydroxide, calcium hydroxide, magnesium hydroxide, zinc hydroxide. Organic bases suitable for preparation of the disclosed acid salts are amines and amino acids of the sufficient basicity to produce a stable salt and suitable for use for medical purposes (in particular, they are to have low toxicity). Such amines include ammonia, methylamine, dimethylamine, trimethylamine, ethylamine, diethylamine, triethylamine, benzylamine, dibenzylamine, dicyclohexylamine, piperazine, ethylpiperidine, tris(hydroxymethyl)aminomethane and the like. Besides, salts can be prepared using some tetraalkylammonium hydroxides, such as holine, tetramethylammonium, tetraethylammonium, and the like. Aminoacids may be selected from the main aminoacids - lysine, ornithine and agrinine.
**"Fragment"** (scaffold) means the structural formula of a part of the molecule characteristic of a group of compounds in the "combinatorial library".
**"1,2-Ethylene radical"** means -CH₂-CH₂- group comprising one or more "alkyl substituents" of the same or different structure the meanings of which are defined in this section.

The subject of the present invention is substituted 8-sulfonyl-2,3,4,5-tetrahydro-1*H-γ-*carbolines of the general formula 1 and pharmaceutically acceptable salts thereof, 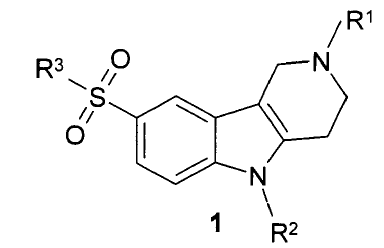 wherein:
**R¹** represents a substituent selected from hydrogen; C₁-C₃ alkyl, optionally substituted phenyl; C₁-C₄ alkoxycarbonyl;
**R²** represents a substituent of cyclic system selected from hydrogen; C₁-C₃ alkyl optionally substituted with phenyl, pyridin-(3- or 4-yl), (6-methylpyridin-3-yl); C₂-C₃ alkenyl substituted with phenyl; optionally substituted phenylsulfonyl;
**R³** represents phenyl optionally substituted with halogen; 6-membered aromatic azaheterocyclyl; C₁-C₃ dialkylamino group; phenylamino group, in which phenyl is optionally substituted with halogen; saturated 6-membered azaheterocyclyl comprising additional nitrogen atom substituted with C₁-C₃ alkyl.

The preferred substituted 8-sulfonyl-2,3,4,5-tetrahydro-1*H*-γ-carbolines of the general formula **1** are 8-arylsulfonyl-2,3,4,5-tetrahydro-1*H*-γcarbolines of the general formula **1.1** and amides of 2,3,4,5-tetrahydro-1*H*-γ-carboline-8-fulfonic acid of the general formula **1.2** and pharmaceutically acceptable salts thereof, 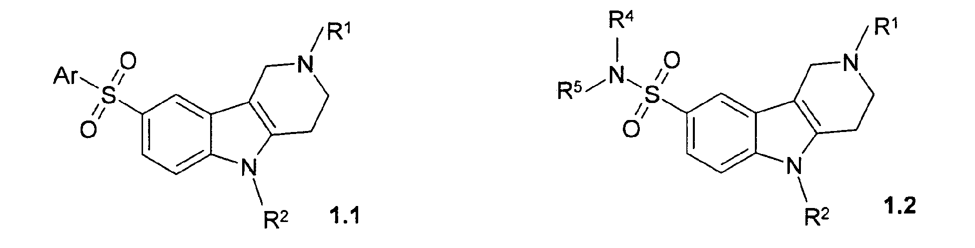 wherein:
**R¹** and **R²** are all as mentioned above for the compounds of the general formula **1**;
Ar represents phenyl optionally substituted with halogen or 3- or 4- pyridyl,
**R⁴** and **R⁵** independently of each other represent hydrogen, always supposing that they both are not hydrogen at the same time; C₁-C₃ alkyl; phenyl optionally substituted with halogen; or **R⁴** and **R⁵** together with the nitrogen atom to which they are attached form 4-alkyl substituted piperazin-1-yl.

The preferred substituted 8-sulfonyl-2,3,4,5-tetrahydro-1*H*-γ-carbolines of the general formulas **1.1** and **1.2** are substituted 8-phenylsulfonyl-2,3,4,5-tetrahydro-1*H-γ*-carbolines of the general formula **1.1.1** and substituted phenyl amides of 2,3,4,5-tetrahydro-1*H*-γ-carbolin-8-sulfonic acid of the general formula **1.2.1** and pharmaceutically acceptable salts thereof, 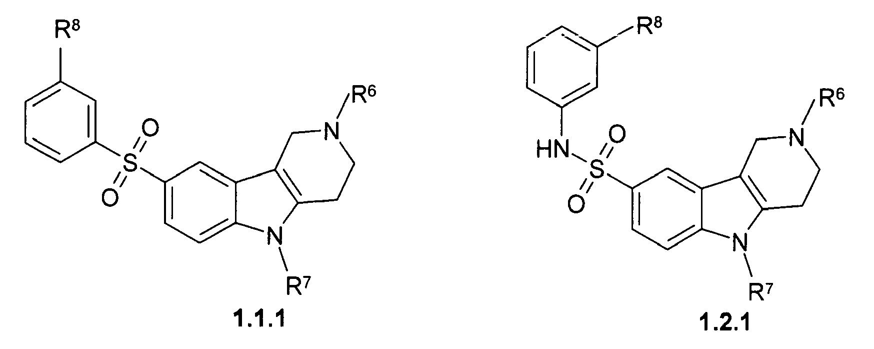 wherein:
**R⁶** and **R⁷** independently of each other represent hydrogen or methyl;
**R⁸** represents hydrogen, chloro or fluoro.

The preferred substituted 8-sulfonyl-2,3,4,5-tetrahydro-1*H*-γ-carbolines of the general formulas **1.1.1** and **1.2.1** are substituted 8-phenylsulfonyl-2,3,4,5-tetrahydro-1*H*-γ-carbolines of formulas **1.1.1(1), 1.1.1(2), 1.1.1(3),1.1.1(4), 1.1.1(5), 1.1.1(6), 1.1.1(7), 1.1.1(8), 1.1.1(9)** and substituted phenyl amides of 2,3,4,5-tetrahydro-1*H*-γ-carboline-8-sulfonic acid of formulas **1.2.1(1), 1.2.1(2), 1.2.1(3), 1.2.1(4), 1.2.1(5), 1.2.1(6), 1.2.1(7), 1.2.1(8), 1.2.1(9)** and pharmaceutically acceptable salts thereof,

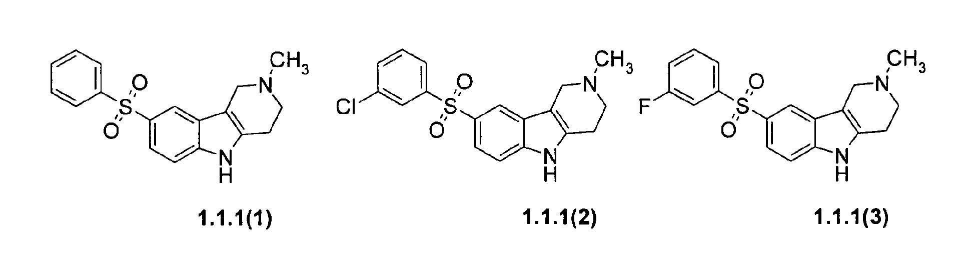

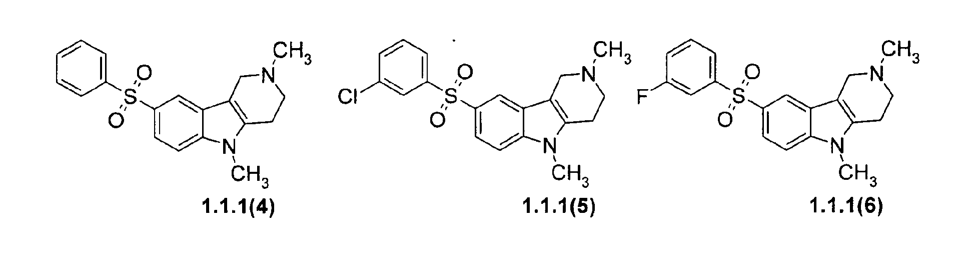

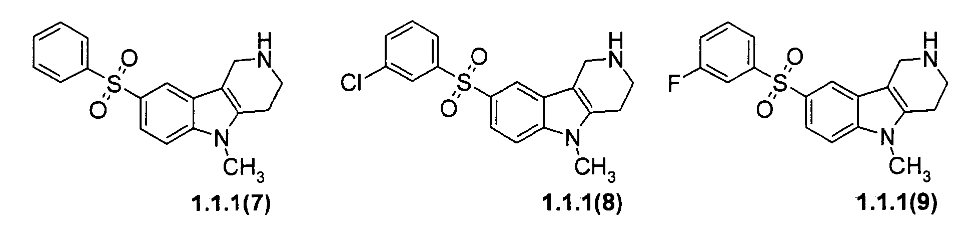

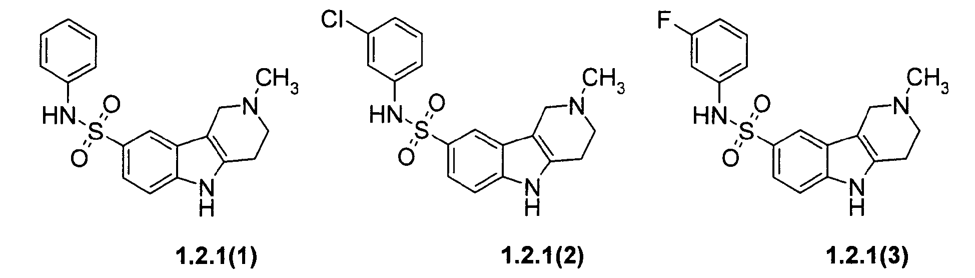

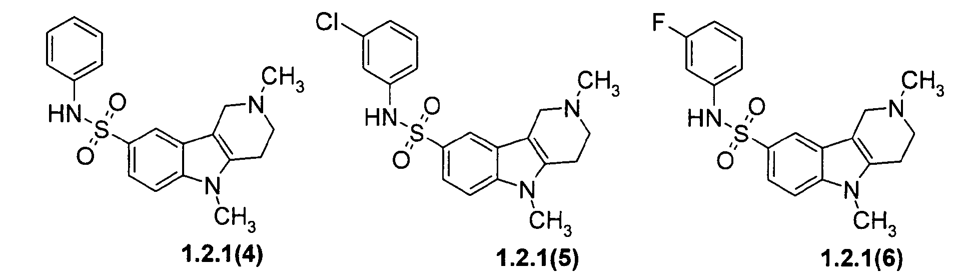

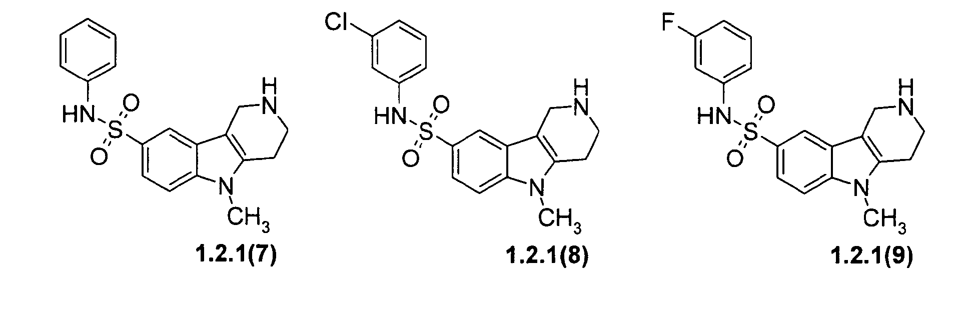

The subject of the present invention is a method for preparation of 8-arylsulfonyl-2,3,4,5-tetrahydro-1*H*-γ-carbolines of the general formula **1.1** by interaction of 8-bromo-2,3,4,5-tetrahydro-1*H*-γ-carbolines of the general formula **2** with sulfinic acids of the general formula **3** in the presence of cuprous iodide, N,N'-dimethylethylenediamine and base in aprotonic solvent media according to the scheme given below: 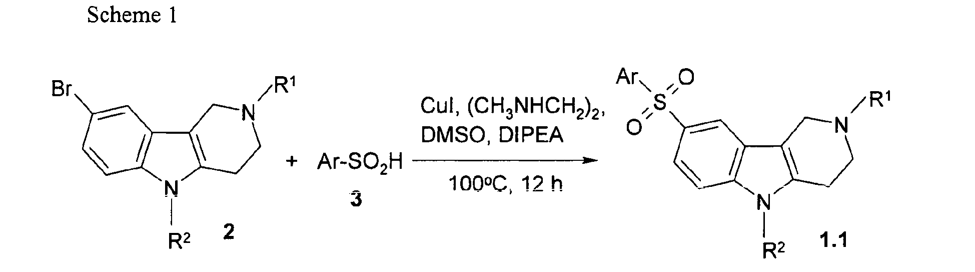 wherein:
**R¹, R²** and **Ar** are all as mentioned above, DIPEA means N,N'-diisopropylethylamine.

The subject of the present invention is also a method for preparation of 8-arylsulfonyl-2,3,4,5-tetrahydro-1*H*-γ-carbolines of the general formula **1.1** by interaction of 2,3,4,5-tetrahydro-1*H*-γ-carboline-8-sulfinic acid of the general formula **4** with aryl iodides of the general formula 5 in the presence of cuprous iodide, N,N'-dimethylethylenediamine and base in aprotonic solvent media according to the scheme given below: 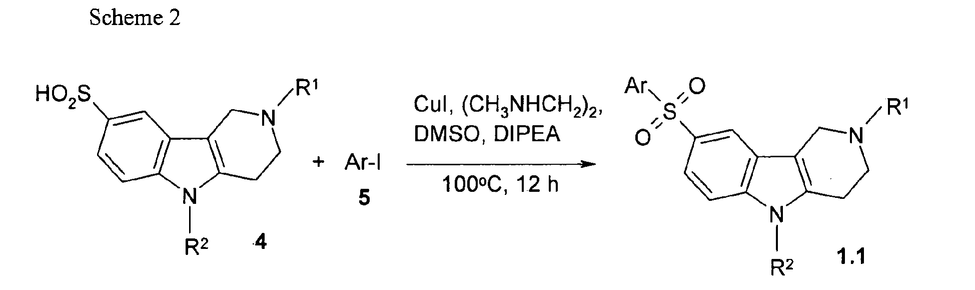 wherein:
**R¹, R²** and **Ar** have the above meanings.

The subject of the present invention is also a method for preparation of 8-arylsulfonyl-2,3,4,5-tetrahydro-1*H*-γ-carbolines of the general formula **1.1.3** at subsequent action of sodium hydride and alkylating agent (R²-Br 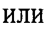 R²-I) in DMF on 8-arylsulfonyl-2,3,4,5-tetrahydro-1*H-*γ-carbolines of the general formula **1.1.2** according to the scheme given below: 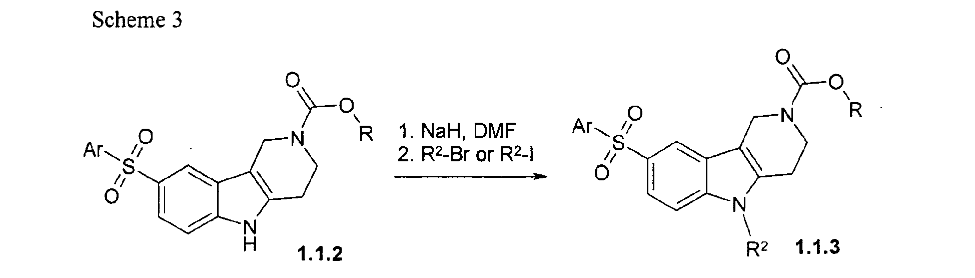 wherein:
**R²** and **Ar** have the above meanings;
**R** represents ethyl or *tert.* -butyl.

The subject of the present invention is also a method for preparation of 8-arylsulfonyl-2,3,4,5-tetrahydro-1*H*-γ-carbolines of the general formula **1.1.4** by the action of alcali on 2-ethoxycarbonyl-8-arylsulfonyl-2,3,4,5-tetrahydro-1*H*-γ-carbolines of the general formula **1.1.3,** in which R=Et, or hydrogen chloride on 2-*tert*.-butyloxycarbonyl-8-arylsulfonyl-2,3,4,5-tetrahydro-1*H*-γ-carbolines of the general formula **1.1.3,** in which R=t-Bu, according to the scheme given below: 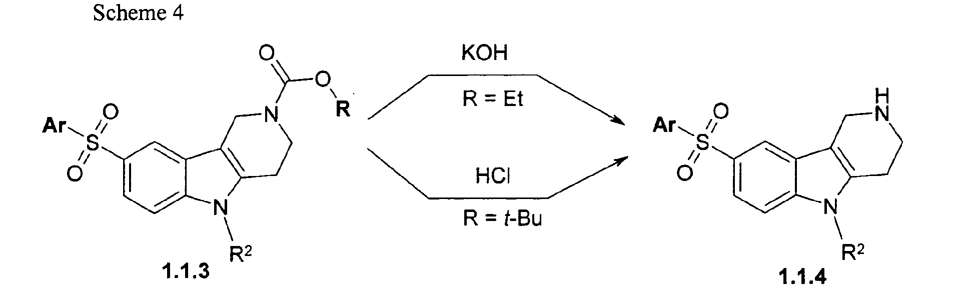 wherein:
**R²** and **Ar** have the above meanings;
**R** represents ethyl or *tert.* -butyl.

The subject of the present invention is also a method for preparation of 8-arylsulfonyl-2,3,4,5-tetrahydro-1*H*-γ-carbolines of the general formula **1.1.1** by reductive amination of aldehydes (R¹-CHO) with 8-arylsulfonyl-2,3,4,5-tetrahydro-1*H*-γ-carbolines of the general formula **1.1.4** consisting in interaction of γ-carbolines **1.1.4** with aldehydes and subsequent reduction by NaBH(OAc)₃, according to the scheme given below: 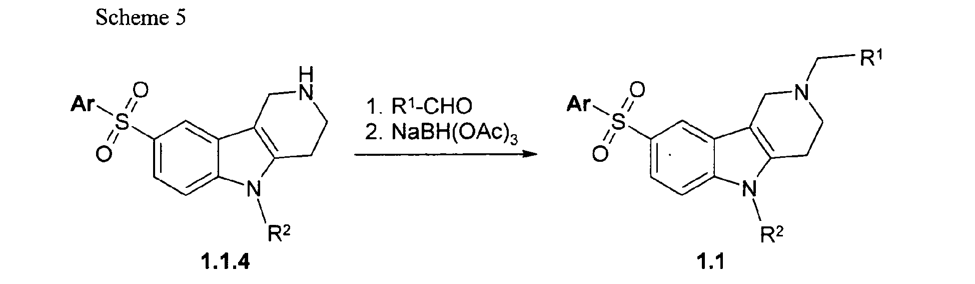 wherein:
**R¹, R²** and **Ar** have the above meanings.

The subject of the present invention is also a method for preparation of 2,3,4,5-tetrahydro-1*H*-γ-carboline-8-sulfonic acid amides of the general formula **1.2** by interaction of 2,3,4,5-tetrahydro-1*H*-γ-carboline-8-sulfonyl chlorides of the general formula **6** with amines of formula **7** in the presence of base, for example, pyridine, according to the scheme given below: 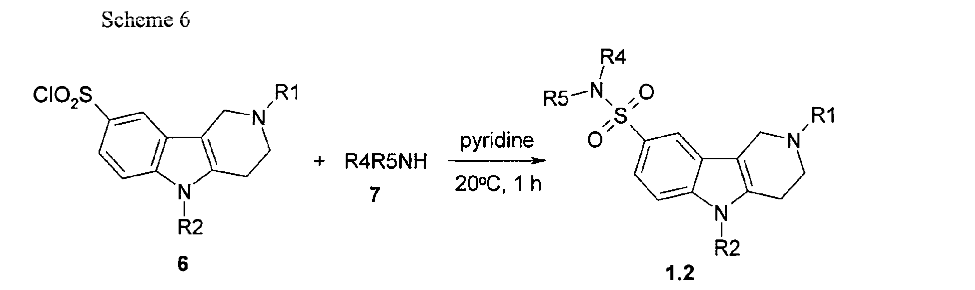 wherein:
**R¹, R², R⁴** and **R⁵** have the above meanings.

The subject of the present invention is also a method for preparation of 2,3,4,5-tetrahydro-1*H*-γ-carboline-8-sulfonic acid amides of the general formula **1.2.3** by the action of hydrogen chloride on amides of 2-*tert*.-butyloxycarbonyl-2,3,4,5-tetrahydro-1*H*-γ-carboline-8-sulfonic acids of the general formula **1.2.2** according to the scheme given below: 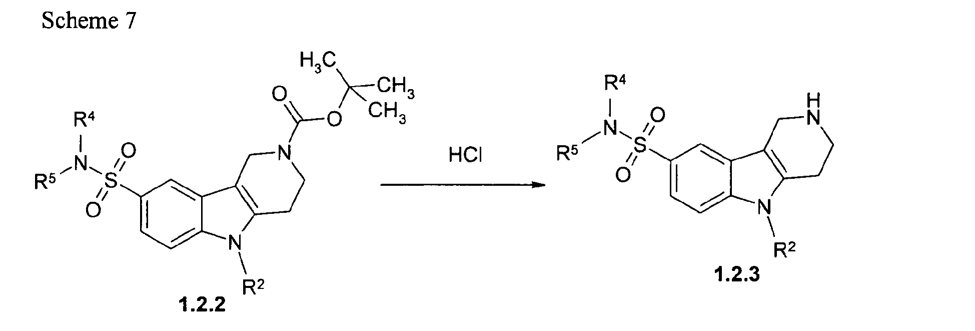 wherein:
**R², R⁴** and **R⁵** have the above meanings.

The subject of the present invention is also a method for preparation of 2,3,4,5-tetrahydro-1*H*-γ-carboline-8-sulfonic acid amides of the general formula **1.2** by reductive amination of aldehydes (R¹-CHO) with amides of 2,3,4,5-tetrahydro-1*H*-γ-carboline-8-sulfonic acids of the general formula **1.2.3,** consisting in interaction of γ-carbolines **1.2.3** with aldehydes and subsequent reduction by NaBH(OAc)₃ according to the scheme given below: 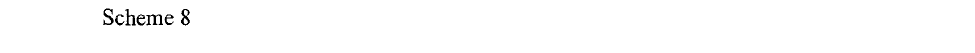 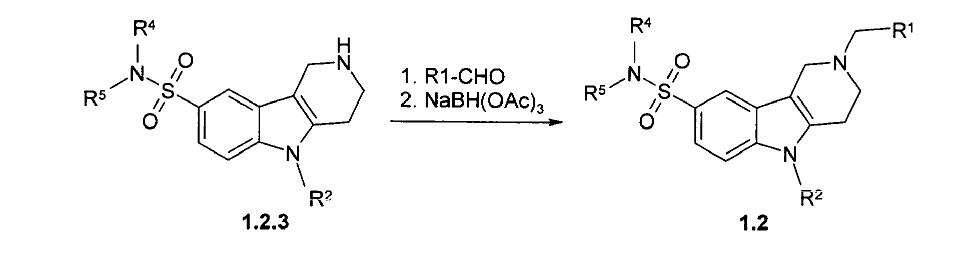 wherein:
**R¹, R², R⁴** and **R⁵** have the above meanings.

The subject of the present invention is also a method for preparation of substituted 5-vinyl-8-sulfonyl-2,3,4,5-tetrahydro-1*H*-γ-carbolines of the general formula **1.4** by interaction of substituted 8-sulfonyl-2,3,4,5-tetrahydro-1*H*-γ-carbolines of the general formula **1.3** with arylacetylenes of the general formula **8** in DMSO in the presence of water alkali and tetrabutylammonium chloride (TBAC) according to the scheme given below: 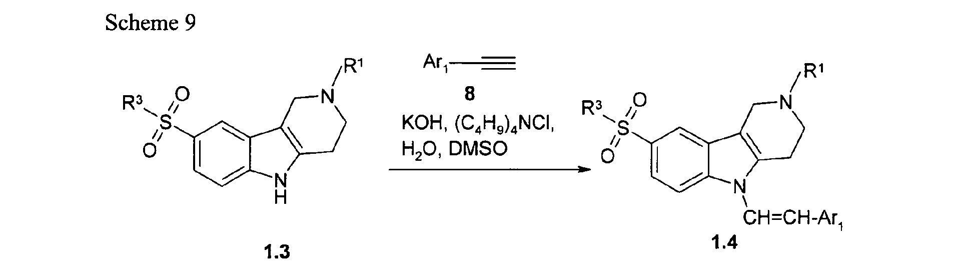 wherein:
**R¹** and **R³** have the above meanings;
**Ar₁** represents optionally substituted aryl or optionally substituted heterocyclyl.

The subject of the present invention is also a method for preparation of substituted 5-ethyl-8-sulfonyl-2,3,4,5-tetrahydro-1*H*-γ-carbolines of the general formula **1.5** by catalytic reduction of substituted 5-vinyl-8-sulfonyl-2,3,4,5-tetrahydro-1*H*-γ-carbolines of the general formula **1.4** with hydrogen according to the scheme given below: 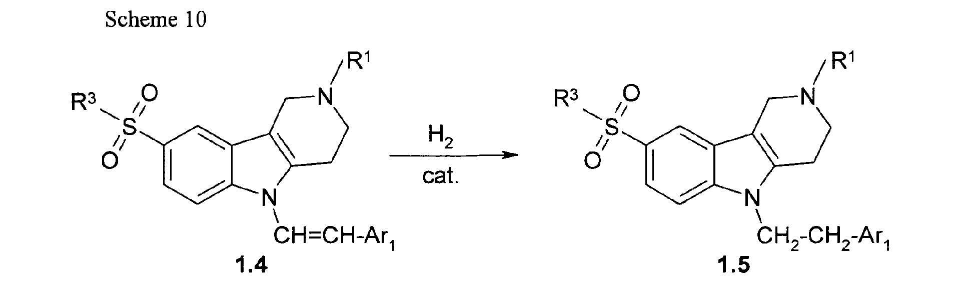 wherein:
**R¹, R³** and **Ar₁** have the above meanings.

The subject of the present invention is also a method for preparation of substituted 5-(2-pyridinylethyl)-8-sulfonyl-2,3,4,5-tetrahydro-1*H*-γ-carbolines of the general formula **1.6** by interaction of substituted 8-sulfonyl-2.3.4,5-tetrahydro-1*H*-γ-carbolines of the general formula **1.3** with vinylpyridines of the general formula **9** in DMSO in the presence of water alkali and tetrabutylammonium chloride according to the scheme given below: 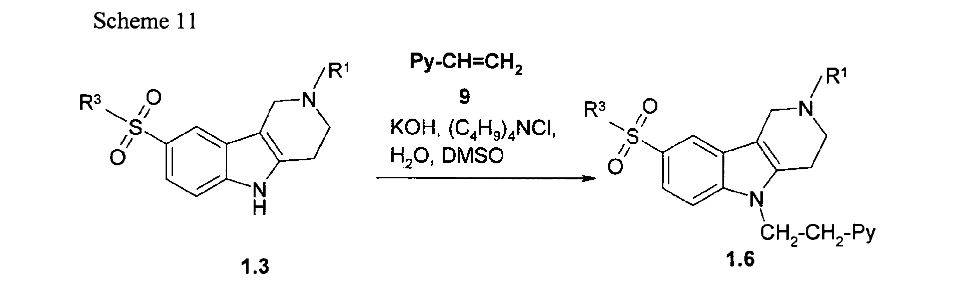 wherein:
**R¹** and **R³** have the above meanings;
**Py** represents optionally substituted 3- or 4- pyridinyl.

The starting sulfinic acids **4** and sulfonyl chlorides **6** were prepared using well known transformations according to the following scheme: 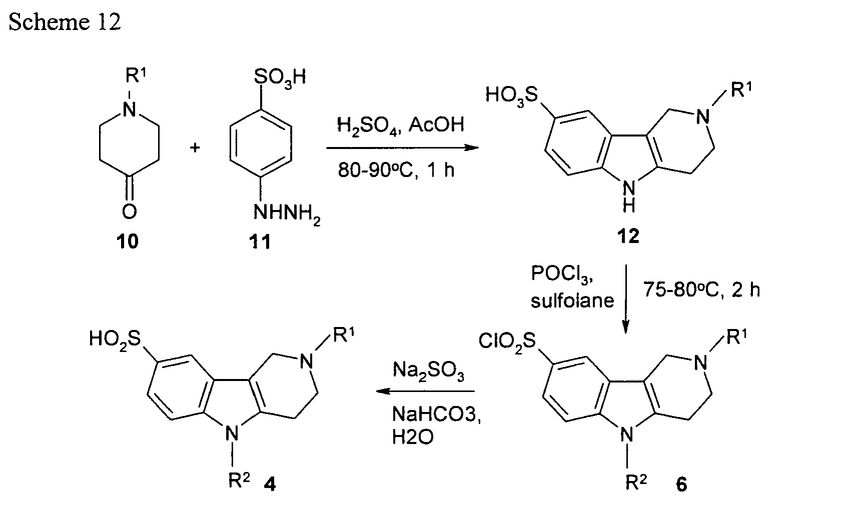 wherein:
**R¹** and **R²** have the above meanings.

The subject of the present invention is novel ligands, the range of biological activity of which simultaneously includes alpha-adrenoceptors, dopamine receptors, histamine receptors, imidazoline receptors, sigma receptors, norepinefrine receptors, serotonin receptors, representing substituted 8-sulfonyl-2,3,4,5-tetrahydro-1*H*-γ-carbolines of the general formula **1** in the form of free bases or pharmaceutically acceptable salts.

The subject of the present invention is novel ligands exhibiting antagonistic activity towards serotonin 5-HT₆ receptors and representing substituted 8-sulfonyl-2,3,4,5-tetrahydro-1*H*-γ-carbolines of the general formula **1** in the form of free bases or pharmaceutically acceptable salts.

The subject of the present invention is an active component for pharmaceutical compositions and medicaments intended for treatment and prophylaxis of pathologic conditions and diseases of CNS, representing a ligand of the general formula **1** in the form of free bases or pharmaceutically acceptable salts.

The subject of the present invention is a pharmaceutical composition, the range of biological activity of which simultaneously includes alpha-adrenoceptors, dopamine receptors, histamine receptors, imidazoline receptors, sigma receptors, norepinefrine receptors, serotonin receptors, comprising as an active component therapeutically effective amount of ligand of the general formula **1** or its pharmaceutically acceptable salt.

Pharmaceutical compositions may include pharmaceutically acceptable excipients. Pharmaceutically acceptable excipients mean diluents, auxiliary agents and/or carriers applied in the sphere of pharmaceutics. According to the invention pharmaceutical composition together with an active component of the general formula **1** may include other active ingredients provided that they do not give rise to undesirable effects, such as allergic reactions.

If needed, according to the present invention pharmaceutical compositions could be used in clinical practice in various forms prepared by mixing the said compositions with traditional pharmaceutical carries, for example, peroral forms (such as, tablets, gelatinous capsules, pills, solutions or suspensions); forms for injections (such as, solutions or suspensions for injections, or a dry powder for injections which requires only addition of water for injections before utilization); local forms (such as, ointments or solutions).

According to the present invention the carriers used in pharmaceutical compositions represent carriers which are used in the sphere of pharmaceutics for preparation of commonly used forms. Binding agents, greasing agents, disintegrators, solvents, diluents, stabilizers, suspending agents, colorless agents, taste flavors are used for peroral forms; antiseptic agents, solubilizers, stabilizers are used in forms for injections; base materials, diluents, greasing agents, antiseptic agents are used in local forms.

The subject of the present invention is also a method for preparation of pharmaceutical composition consisting in mixing of at least one active component of the general formula 1 or its pharmaceutically acceptable salt with inert filler and/or solvent.

The subject of the present invention is also a medicament in the form of tablets, capsules, or injections, placed in pharmaceutically acceptable packing, including a novel active component or novel pharmaceutical composition intended for treatment and prophylaxis of pathologic conditions and diseases of CNS.

Pathologic conditions and diseases of CNS include, but are not restricted by hyperkinetic disorders among them imbecility, anxiety disorders, psychic disorders and schizophrenia, depression, cognitive disorders or cognitive functions disturbance, inter alia Alzheimer's disease and Huntington's disease, neurodegenerative diseases, chemical substance dependence, disorders caused by chemical substances and so on.

The subject of the present invention is also a method for treatment of pathologic conditions and diseases of CNS by administration of therapeutically effective amount of active component of the general formula **1** or its pharmaceutically acceptable salt, or medicament, or pharmaceutical composition comprising the said active component.

Clinical dose of pharmaceutical composition or medicament comprising as an active component ligands of the general formula **1** may be corrected depending on: therapeutic efficiency and bio-accessibility of active ingredients in patients' organism, rate of their exchange and removal from organism, and age, gender, and severity of patient's symptoms. Thus, the daily intake for adults normally being 10∼500 mg, preferably 50-300 mg. Accordingly, the above effective doses are to be taken into consideration while preparing medicament of the present invention, each dose unit of the medicament contains 10∼500 mg of of a ligand of general formula 1, preferably 50∼300 mg. Following the instructions of physician or pharmacist, the medicaments may be taken several times over specified periods of time (preferably, from one to six times).

### Best Embodiment of the invention

The invention is illustrated by the following figures:

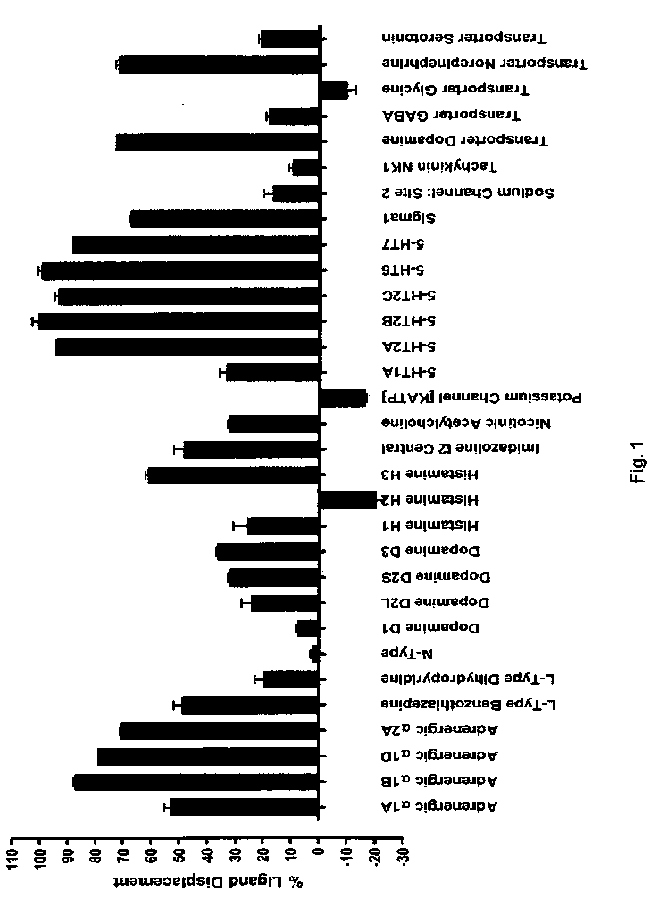

**Fig. 1** The range of pharmacological activity of 2,5-dimethyl-8-(phenylsulfonyl)-2,3,4,5-tetrahydro-1*H*-γ-carboline hydrochloride **1.1.1**(4)·HCl (interaction with 31 receptors). The interaction was estimated by the displacement of radio-labeled ligans from their complex with the corresponding receptors by compound **1.1.1**(4)·HCl at its concentration of 10 mkM.

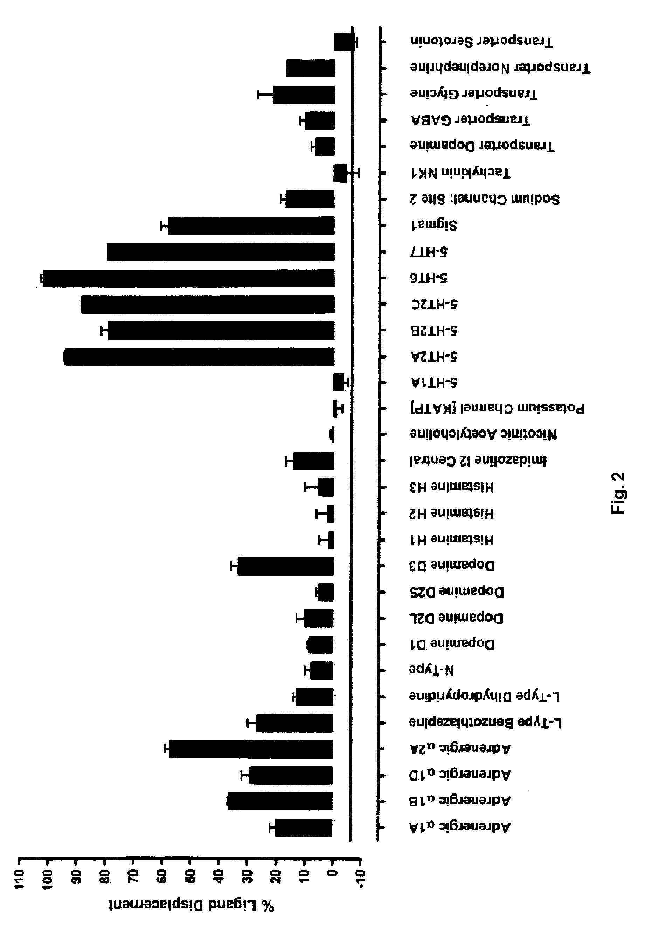

**Fig. 2** The range of pharmacological activity of 2-methyl-2,3,4,5-tetrahydro-1*H*-γ-carboline-8-sulfonic acid 3-fluorophenyl amide hydrochloride **1.2.1**(3)·HCl (interaction with 31 receptors). The interaction was estimated by the displacement of radio-labeled ligans from their complex with the corresponding receptors by compound **1.2.1**(3)·HCl at its concentration of 1 mkM.

Below the invention is described by means of specific examples, which illustrate but not limit the scope of the invention.

**Example 1. 2,3,4,5-Tetrahydro-1*H*-γ-carboline-8-sulfonic acids 12(1-4) (general method, scheme 12).** A mixture of piperidone of formula **10** (1-4) (0.10 mol) and 4-hydrazinobenzenesulfonic acid 11 (18.8 g, 0.10 mol) in AcOH (200 ml) was boiled for 2 h. The precipitate formed after cooling the mixture was filtered off, washed with AcOH, *i*-PrOH, and hexane. It was dryed in the opened air. H₂SO₄ (50 ml) was added to suspension of the prepared product in AcOH (140 ml) and the resultant mixture was stirred at 80-90°C for 1 h. After cooling the mixture was diluted with AcOH (200 ml) and poured into ether (1.5 1) at vigorous stirring. The solvent was decanted, the residue was twice washed with ether, treated with boiling methanol and left in freezer for night. Obtained precipitate of 2,3,4,5-tetrahydro-1*H*-γ-carboline-8-sulfonic acid **12**(1-4) hydrosulfate was filtered off, washed with methanol, ether and dryed in *vacuo.* Compounds **12**(1-4) were prepared as hydrosulfates, yield 59-74%: LCMS 2-methyl-2,3,4,5-tetrahydro-1*H*-γ-carboline-8-sulfonic acid **12**(1) hydrosulfate - *m*/*z*: 267 (M+H)⁺; 2-benzyl-2,3,4,5-tetrahydro-1*H*-γ-carboline-8-sulfonic acid **12**(2) hydrosulfate - *m*/*z*: 343 (M+H)⁺; 2-ethyloxycarbonyl-2,3,4,5-tetrahydro-1*H*-γ-carboline-8-sulfonic acid **12**(3) hydrosulfate - *m*/*z*: 325 (M+H)⁺; 2-*tert*.-butyloxycarbonyl-2,3,4,5-tetrahydro-1*H*-γ-carboline-8-sulfonic acid **12**(4) hydrosulfate - *m*/*z:* 353 (M+H)⁺.

**Example 2. 2,3,4,5-Tetrahydro-1*H*-γ-carboline-8-sulfonyl chlorides 6(1-4) (general method, scheme 12).** A mixture of sulfonic acid **12**(1-4) (20 mmol) and POCl₃ (4.0 ml, 6.58 g, 43 mmol) in sulfolane (30 ml) was stirred at 75-80°C for 2 h. The prepared solution was poured into dry ether (0.5 1) at vigorous stirring, the ether layer was decanted, the residue was washed twice with dry ether and treated with EtOAc. The suspension was stirred for 12 hs, the formed crystalline precipitate was filtered off, washed with EtOAc, ether and dryed in *vacuo.* Hydrosulfates of sulfonyl chlorides **6**(1-4) were prepared (Table 3). They were used for preparation of compounds of the general formulas **1.1** and **1.2** without additional purification.

**Table 3. LCMS data for 2,3,4,5-tetrahydro-1H-γ-carboline-8-sulfonyl chlorides 6(1-4).**

| **N° comp.** | **R¹** | **R²** | **LCMS, *m*/*z*: (M+1)⁺** |
|---|---|---|---|
| **6**(1) | CH₃ | H | 285 |
| **6**(2) | Ph-CH₂ | H | 361 |
| **6**(3) | C₂H₅OCO | H | 461 |
| **6**(4) | (CH₃)₃COCO | H | 371 |

**Example 3. 8-(Arylsulfonyl)-2,3,4,5-tetrahydro-1*H*-γ-earbolines 1.1(1-3,6) and 1.1.1(1-3) (general method, schemes 12, 2).** Sulfonyl chloride **6**(1-4) (1.28 g, 3.35 mmol) was added to a vigorously stirred saturated Na₂SO₃ solution (20 ml). Then, NaHCO₃ (3.36 g, 40 mmol) was added in three portions and the resultant mixture was stirred for 1 h at 80-85°C. The mixture was neutralized with AcOH, evaporated to dryness at reduced pressure, treated with methanol and filtered off. The precipitate was washed with icy water, methanol and dryed in *vacuo.* 2-Methyl-2,3,4,5-tetrahydro-1*H*-γ-carboline-8-sulfinic acids **4**(1-5) were prepared, yield 46-62%. LCMS spectra of the compounds showed the corresponding molecular ions. Acids **4**(1-5) were used in further transformations without additional purification. CuI (26 mg, 0.14 mmol) was added to stirred solution of *N*,*N*'-dimethylethylenediamine (24 mg, 0.27 mmol) in DMSO (2.0 ml), the resultant mixture was stirred for 10 min, then water (0.7 ml), sulfinic acid **4**(1-5) (1.50 mmol), DIPEA (0.26 ml, 1.50 mmol), and the corresponding (het)aryl iodide 5 (1.50 mmol) were added successively. The reaction vessel was blown through with argon, plugged up tightly, and the reaction mixture was stirred at 100°C by night, then it was cooled and treated with water. The precipitate formed was separated by centrifugation, successively washed with water, saturated solution of NaHCO₃, aqua ammonia and water, every time separating the solid by centrifugation. The compounds **1.1**(1-3,6) and **1.1.1**(1-3) presented in Table 4 were prepared, yield 18-23%. Hydrochlorides of **1.1** were prepared by the addition of 10% excess (100 mg/ml) of hydrogen chloride solution in dioxane to solution of the corresponding base in acetone.

**Example 4. Substituted 5-alkyl-8-(arylsulfonyl)-2-ethoxycarbonyl-2,3,4,5-tetrahydro-1*H*-γ-carbolines 1.1(4-5), 1.1(7-9) (general method, scheme 3).** NaH (1.43 g of 50-60% oil suspention) was added to a previously cooled to -10°C solution of 2-ethoxycarbonyl-2,3,4,5-tetrahydro-1*H*-γ-carboline **1.1.2** (10 mmol) in DMFA (25 ml), in argon atmosphere and at stirring; the mixture was stirred untill hydrogen evolution (30-45 min) was completed, then a solution of alkylating agent (12 mmol), for example, methyl iodide or benzyl chloride in DMFA (5 ml) was added dropwise to the reaction mixture. The mixture was stirred at the above temperature for 1 h, then slowly warmed to room temperature, stirred (about 1 h) untill the conversion of the starting γ-carboline (LCMS control) was completed and transferred onto ice-water mixture. The product was extracted with CHCl₃, the combined organic layers were successively washed with water, 5% aqua HCl, water, saturated NaCl solution, dryed over Na₂SO₄, evaporated at reduced pressure; the residue was subjected to flash-chromatography (hexane - AcOEt), N-alkylated compounds **1.1**(5-9) presented in Table 4, were prepared, yield 65 - 90%.

**Example 5. Substituted 5-alkyl-8-(arylsulfonyl)-2,3,4,5-tetrahydro-1*H*-γ-carbolines 1.1.1(7-9) (general method, scheme 4).** KOH (16.8 g, 30 mmol) solution in 85% aqua ethanol was added to a suspension of 5-methyl-8-(arylsulfonyl)-2-ethoxycarbonyl-2,3,4,5-tetrahydro-1*H*-γ-carboline 1.1(7-9) (10 mmol) in ethanol (20 ml), in inert atmosphere and at stirring; the resultant mixture was boiled at vigorous stirring within 24 hs, evaporated at reduced pressure, the residue was treated with water, extracted with CHCl₃, the combined organic layers were washed with water, saturated NaCl solution, dryed over Na₂SO₄, evaporated at reduced pressure, γ-carbolines 1.1.1(7-9), presented in Table 4, were prepared, yield 72-80%.

**Example 6. 5-Methyl-8-(sulfonyl)-2,3,4,5-terahydro-1*H*-pyrido[4,3-b]indoles 1.1(7-9) and 1.1.1(4-6) (general method, scheme 1).** CuI (26 mg, 0.14 mmol) was added to a stirred solution of *N*,*N*'-dimethylethylenediamine (24 mg, 0.27 mmol) in DMSO (2.0 ml), the resultant mixture was stirred for 10 min, then water (0.7 ml) and the corresponding sodium sulphinate **3** (1.50 mmol), DIPEA (0.26 ml, 1.50 mmol) and 8-bromo-γ-carboline **2**(1-3) (1.50 mmol) were successivly added to it. The reaction vessel was blown through with argon, plugged up tightly, and the reaction mixture was stirred at 100°C, then it was cooled and treated with water. The precipitate formed was separated by centrifugation, successively washed with water, saturated NaHCO₃ solution, aqua ammonia and water, every time separating the solid by centrifugation. The product was separated by chromatography on silica gel impregnated with triethylamine (eluent: hexane - EtOAc - Et₃N). Sulfones **1.1**(7-9) and **1.1.1**(4-6) represented in Table 4 were prepared, yield 23-34%. Hydrochlorides thereof were prepared by the addition of 10% excess (100 mg/ml) of hydrogen chloride solution in dioxane to the solution of the corresponding base in acetone.

**Example 7. 2-Methyl-5-(4-methylphenylsulfonyl)-8-(phenylsulfonyl)-2,3,4,5-tetrahydro-1*H*-γ-caboline hydrochloride 1.1(10),(scheme 3).** NaH (96 mg, 60% oil suspension, 2.4 mmol) was washed with hexane; at external cooling with ice, dry DMF (10 ml) and γ-carboline **1.1.1**(1) (2 mmol) were added to it. After hydrogen evolution (30 min) was completed, p-toluene sulfonyl chloride (2.4 mmol) was added and the mixture was stirred for 30 min at 20°C. The reaction mixture was poured into water; the precipitated solid was filtered off, washed with 10% K₂CO₃ solution, water and dryed. It gave γ-carboline **1.1**(10), yield 85% (Table 4), which was converted into hydrochloride by mixing 10% excess (100 mg/ml) of hydrogen chloride solution in dioxane with solution of **1.1**(10) base in acetone.

**Example 8. 2-Methyl-5-styryl-8-(phenylsylfonyl)-2,3,4,5-tetrahydro-1*H*-γ-carboline hydrochloride 1.1(11) (scheme 9) and 2-methyl-5-phenethyl-8-(phenylsulfonyl)-2,3,4,5-tetrahydro-1*H*-γ-carboline hydrochloride 1.1(12) (scheme 10).** TBAC (100 mkl, 50% solution), freshly distilled phenylacetylene (0.32 g, 3 mmol) and KOH (8 ml, 60% solution) were added to the solution γ-carboline **1.1.1**(1) (0.68 g, 2 mmol) in DMSO (2 ml) successivly. The reaction mixture was stirred vigorously in argon atmosphere for 12 hs at 60°C, then CH₂Cl₂ was added until solution obtained, the resultant solution was washed with NaCl solution and dryed over Na₂SO₄. The solvent was evaporated to dryness, the residue was subjected chromatogaphy on silica gel, it gave 2-methyl-5-styryl-8-(phenylsulfonyl)-2,3,4,5-tetrahydro-1*H*-γ-carboline hydrochloride **1.1**(11), yield 68%. The prepared product was reduced by hydrogen over PtO₂ at room temperature in ethanol at 1 atm. It gave 2-methyl-5-phenethyl-8-(phenylsulfonyl)-2,3,4,5-tetrahydro-1*H*-γ-carboline 1.1(12), yield 95% (Table 4), which was converted into hydrochloride by mixing 10% excess (100 mg/ml) of hydrogen chloride solution in dioxane with solution of 1.1(12) base in acetone.

**Example 9 2-Methyl-5-(2-(pyridinyl)ethyl)-8-(phenylsulfonyl)-2,3,4,5-tetrahydro-1*H*-pyrido[4,3-*b*]indole hydrochlorides 1.1(13-15), (scheme 11).** TBAC (100 mkl, 50% solution), freshly distilled corresponding 2-vinylpyridine (0.32 g, 3 mmol) and KOH (8 ml, 60% solution) were added to the solution γ-carboline **1.1.1**(1) (0.68 g, 2 mmol) in DMSO (2 ml) successivly. The reaction mixture was stirred vigorously in argon atmosphere for 12 hs at 60°C, then CH₂Cl₂ was added until solution obtained, the resultant solution was washed with NaCl solution and dryed over Na₂SO₄. The solvent was evaporated to dryness at reduced pressure, the residue was subjected chromatogaphy on silica gel, it gave γ-carbolines **1.1**(13-15), yield 68-76% (Table 4), which were converted into hydrochlorides by mixing 10% excess (100 mg/ml) of hydrogen chloride solution in dioxane with solution of the corresponding base in acetone.

**Table 4. Results LCMS and ¹H NMR data for the spectra 8-(arylsulfonyl)-2,3,4,5-tetrahydro-1H-γ-carbolines of the general formula 1.1.**

| **N° comp.** | **R¹** | **R²** | **Ar** | **LCMS, *m*/*z*: (M+1)⁺** | **¹H NMR (DMSO-d₆, 400 MHz), δ, m.d. (*J*, Hz)** |
|---|---|---|---|---|---|
| **1.1**(1) | CH₃ | H | 3-Py | 328 | 2.92 (d, *J* = 4.8, 3H), 3.07 (br. m, 1H), 3.24 (m, 1H), 3.44 (m, 1H), 3.67 (br. m, 1H), 4.29 (d. d, *J₁* = 14.4, *J*₂ = 7.6, 1H), 4.66 (d, *J* = 14.4, 1H), 7.55 (d, *J* = 8.4, 1H), 7.62 (d. d, *J₁* = 8.4, *J₂* = 4.0, 1H), 7.69 (d. d, *J₁* = 8.4, *J₂* = 2.0, 1H), 8.25 (d, *J* = 1.6, 1H), 8.31 (d.t, *J₁* = 8.0, *J₂* = 1.6, 1H), 8.79 (d, *J* = 4.8, 1H), 9.11 (d, *J* = 1.6, 1H), 11.34 (br. s, 1H), 12.00 (s, 1H). |
| **1.1**(2) | CH₃ | H | 4-Py | 328 | 2.93 (d, *J* = 4.4, 3H), 3.08 (br. m, 1H), 3.24 (m, 1H), 3.45 (m, 1H), 3.69 (br. m, 1H), 4.30 (d. d, *J₁* = 14.4, *J₂* = 7.6, 1H), 4.68 (d, *J* = 14.4, 1H), 7.58 (d, *J* = 8.4, 1H), 7.67 1H), 7.58 (d, *J* = 1.8, 1H), 7.86 (d. d, *J₁* = 4.4, *J₂* = 1.6, 2H), 8.24 (d, *J* = 1.8, 1H), 8.83 (d. d, *J₁* = 4.4, *J₂ =* 1.6, 2H), 11.17 (br. s, 1H), 12.00 (s, 1H). |
| **1.1**(3) | Ph-CH₂ | H | Ph | 403 | |
| **1.1**(4) | C₂H₅OCO | H | Ph | 385 | |
| **1.1**(5) | C₂H₅OCO | Ph-CH₂ | Ph | 475 | |
| **1.1**(6) | CH₃ | Ph-CH₂ | Ph | 417 | |
| **1.1**(7) | C₂H₅OCO | CH₃ | Ph | 399 | |
| **1.1**(8) | C₂H₅OCO | CH₃ | 3-Cl-C₆H₄ | 433 | |
| **1.1**(9) | C₂H₅OCO | CH₃ | 3-F-C₆H₄ | 417 | |
| **1.1**(10) | CH₃ | 4-CH₃-C₆H₄SO₂ | Ph | 481 | 2.92 (d, *J* = 2.8, 3H), 3.19 (br, 2H), 3.40 (t, *J* = 6.4, 2H), 3.46 (m, 1H), 3.73 (m, 1H), 4.29 (d. d*, J₁ =* 14.4, *J₂* = 6.8, 1H), 4.63-4.69 m, 3H), 7.68 (t, *J* = 6.4, 1H), 7.57-7.64 (m, 4H), 7.73 (d, *J* = 8.8, 1H), 7.78 (d, *J* = 7.2, 1H), 7.92 (d, *J* = 7.2, 2H), 8.18 (s, 1H), 8.20 (t, *J* = 6.4, 1H), 8.70 (d, *J* = 5.2, 1H), 11.40 (br. s, 1H). |
| **1.1**(11) | CH₃ | PhCH=CH | Ph | 429 | |
| **1.1**(12) | CH₃ | PhCH₂CH₂ | Ph | 431 | |
| **1.1**(13) | CH₃ | 4-PyCH₂CH₂ | Ph | 432 | |
| **1.1**(14) | CH₃ | 3-PyCH₂CH₂ | Ph | 432 | |
| **1.1**(15) | CH₃ | 6-CH₃-Py-CH₂CH₂ | Ph | 446 | |
| **1.1.1**(1) | CH₃ | H | Ph | 327 | |
| **1.1.1**(2) | CH₃ | H | 3-Cl-C₆H₄ | 361 | |
| **1.1.1**(3) | CH₃ | H | 3-F-C₆H₄ | 345 | 2.42 (s, 3H), 2.71 (t, *J* = 5.2, 2H), 2.80 (t, *J* = 5.2, 2H), 3.58 (s, 2H), 7.44 (d, *J* = 8.4, 1H), 7.47 (t.d, *J₁* = 8.4, *J₂* = 2.0, 1H), 7.56 (d. d, *J₁* = 8.4, *J₂* = 2.0, 1H), 7.62 (d. t, *J₁* = 9.2, *J*₂ = 8.0, 1H), 7.76 (m, 2H), 8.05 (d, *J* = 1.2, 1H), 11.46 (s, 1H). |
| **1.1.1**(4) | CH₃ | CH₃ | Ph | 341 | 2.93 (s, 3H), 3.18 (br. m, 2H), 3.47 (br. m, 1H), 3.71 (s, 3H), 3.72 (br. m, 1H), 4.32 (br. m, 1H), 4.67 (br. m, 1H), 7.55-7.64 (m, 3H), 7.68 (m, 2H), 7.92 (m, 2H), 8.19 (s, 1H), 11.18 (br. s, 1H). |
| **1.1.1**(5) | CH₃ | CH₃ | 3-Cl-C₆H₄ | 375 | |
| **1.1.1**(6) | CH₃ | CH₃ | 3-F-C₆H₄ | 359 | |
| **1.1.1**(7) | H | CH₃ | Ph | 327 | |
| **1.1.1**(8) | H | CH₃ | 3-Cl-C₆H₄ | 361 | |
| **1.1.1**(9) | H | CH₃ | 3-F-C₆H₄ | 345 | |

**Example 10. *N*,*N*,2-Trimethyl-2,3,4,5-tetrahydro-1*H*-γ-carboline-8-sulfonamide 1.2(1), (scheme 6).** The excess of dimethylamine 7 (2.0 ml, 2M THF solution) was added to a vigorously stirred suspension of 2-methyl-2,3,4,5-tetrahydro-1*H*-γ-carboline-8-sulfonyl chloride **6** (393 mg, 1.0 mmol) in THF (3.0 ml). The reaction mixture was kept in ultrasonic bath for 1 h, the solvent was evaporated in rotary evaporator. The residue was treated with icy water, neutralized with the excess of saturated solution of NaHCO₃, kept for 1 h in ultrasonic bath, the solid was separated by centrifugation, washed twice with water, twice - with isopropyl alcohol, hexane, each time suspending the solid, keeping it in ultrasonic bath and separating the solid by centrifugation. Drying in *vacuo* gave 170 mg (58%) of colorless sulfonamide **1.2**(1) (Table 5).

**Example 11. 2,3,4,5-Tetrahydro-1*H*-γ-carboline-8-sulfonamide 1.2(2-10) and 1.2.1(1-6) (general method, scheme 6).** The corresponding sulfonyl chloride **6** (1 mmol) was added to a vigorously stirred solution of amine **7** (1.05 mmol) in pyridine (2.0 ml). The reaction mixture was kept in ultrasonic bath for 1 h, the solvent was evaporated in *vacuo* and the residue was treated in the same manner as given above for **1.2**(1). It gave sulfonamides **1.2**(2-10) and **1.2.1**(1-6) (Table 5), yield 76-80%. Hydrochlorides were prepared by mixing 10% excess (100 mg/ml) of hydrogen chloride solution in dioxane with the solution of the corresponding base in acetone.

**Example 12. Substituted 5-methyl-2,3,4,5-tetrahydro-1*H*-γ-carboline-8-sulfonamide 1.2.1(7-9) (general method, scheme 4).** A solution of Boc-derivative of formula **1.2**(2-4) (0.5 mmol) in CHCl₃ (1 ml) was treated with of 6M solution of HCl in EtOAc - EtOH (1 ml) (prepared from AcCl and ethanol), the mixture was stirred for 1 h, treated with exccess of ether, the precipitated solid was separated by centrifugation, washed with ether, EtOAc, acetone, each time suspending the solid, keeping it in ultrasonic bath (15 min) and separating the solid by centrifugation. It gave 1*H*-γ-carbilines **1.2.1**(7-9) hydrochlorides (Table 5), yield 85-100 %

**Table 5. Results LCMS and ¹H NMR data for the spectra of amides of 2,3,4,5-tetrahydro-1H-γ-carboline-8-sulfonic acid of the general formula 1.2.**

| **N° comp.** | **R¹** | **R²** | **R⁴** | **R⁵** | **LCMS, *m*/*z*: (M+1)⁺** | **¹H NMR (DMSO-d₆, 400 MHz), δ, m.d. (*J*,Hz)** |
|---|---|---|---|---|---|---|
| **1.2**(1) | CH₃ | H | CH₃ | CH₃ | 294 | 2.40 (s, 3H), 2.52 (s, 6H), 2.69 (t, J= 5.3, 2H), 2.80 (t, *J* = 5.3, 2H); 3.54 (s, 2H), 7.35 (d. d, *J₁*= 8.4, *J₂* = 1.6, 1H), 7.45 (d, *J* = 8.4, 1H), 7.72 (d, *J* = 1.6, 1H), 11.40 (s, 1H). |
| **1.2**(2) | (CH₃)₃COCO | CH₃ | H | Ph | 442 | |
| **1.2**(3) | (CH₃)₃COCO | CH₃ | H | 3-Cl-C₆H₄ | 476 | |
| **1.2**(4) | (CH₃)₃COCO | CH₃ | H | 3-F-C₆H₄ | 460 | |
| **1.2**(5) | Ph-CH₂ | H | H | Ph | 418 | |
| **1.2**(6) | Ph-CH₂ | H | H | 3-F-C₆H₄ | 436 | 2.79 (s, 4H), 3.57 (s, 2H), 3.74 (s, 2H), 6.75 (t. d*, J₁* = 8.4*, J₂* = 1.6, 1H), 6.87 (m, 2H), 7.18 (m, 1H), 7.28 (m, 1H), 7.33-7.42 (m, 6H), 7.76 (s, 1H), 10.26 (s, 1H), 11.38 (s, 1H). |
| **1.2**(7) | (CH₃)₃COCO | H | H | Ph | 428 | |
| **1.2**(8) | (CH₃)₃COCO | Ph-CH₂ | H | Ph | 518 | |
| **1.2**(9) | CH₃ | Ph-CH₂ | H | Ph | 432 | |
| 1.2(10) | CH₃ | H | 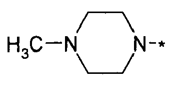 | | 349 | 2.08 (s, 3H), 2.27-2.33 (m, 4H), 2.40 (s 3H), 3.54 (s, 2H), 2.76-2.83 (m, 6H), 2.69 (t, *J* = 5.5, 2H), 7.33 (d. d, *J₁* = 8.6, *J₂* = 1.4, 1H), 7.45 (d, *J* = 8.6, 1H), 7.70 (d, *J* = 1.4, 1H), 11.42 (s,1H). |
| **1.2.1**(1) | CH₃ | H | H | Ph | 342 | |
| **1.2.1**(2) | CH₃ | H | H | 3-Cl-C₆H₄ | 376 | |
| **1.2.1**(3) | CH₃ | H | H | 3-F-C₆H₄ | 360 | 2.90 (d, *J* = 4.5, 3H), 2.98-3.08 (m, 1H), 3.14-3.25 (m, 1H), 3.39-3.44 (m, 1H), 3.62-3.70 (m, 1H), 4.22-4.27 (m, 1H), 4.58-4.66 (m, 1H), 6.72-6.77 (m, 1H), 6.87-6.90 (m, 2H), 7.15-7.21 (m, 1H), 7.46-7.52 (m, 2H), 7.97 (s, 1H), 10.49 (s, 1H), 11.09 (br. s, 1H), 11.86 (s,1H). |
| **1.2.1**(4) | CH₃ | CH₃ | H | Ph | 356 | |
| **1.2.1**(5) | CH₃ | CH₃ | H | 3-Cl-C₆H₄ | 390 | |
| **1.2.1**(6) | CH₃ | CH₃ | H | 3-F-C₆H₄ | 374 | |
| **1.2.1**(7) | H | CH₃ | H | Ph | 342 | |
| **1.2.1**(8) | H | CH₃ | H | 3-Cl-C₆H₄ | 376 | |
| **1.2.1**(9) | H | CH₃ | H | 3-F-C₆H₄ | 360 | |

### Example 13. Determination of antagonistic activity of substituted 8-sulfonyl-2,3,4,5-tetrahydro-1H-γ-carbolines of the general formula 1 towards serotonin 5-HT₆ receptors.

HEK 293 cells with tetracycline-induced T-Rex system (Invitrogen, USA) consistently expressing recombinant human 5-HT₆ receptor, production of Etogen Scientific (USA), were used. The cells were grown in DMEM/10%FBS/1%AAS medium with Blasticidine S and Zeocin (both - Invitrogen, USA). According to the method worked up by the producer of T-Rex system tetracycline hydrochloride was added to the medium with the cells for the purpose of activation of 5-HT₆ receptor expression. The cells were embedded in 96-well plates (30,000-40,000 cells per well) and incubated in 5%-CO₂ incubator at 37°C for 24 hs. Before experiments cells medium was replaced by Hank's balanced salt solution (HBSS), comprising 0,137 M NaCl, 5,4 mM KCl, 0,25 mM Na₂HPO₄, 0,44 mM KH₂PO₄, 1,3 mM CaC1₂, 1,0 mM MgSO₄, 5 MM HEPES (pH 7,4), 0,05% BSA and 1 mM IBMX. The tested compounds of general formula **1** were added to the cells in various concentrations. After incubation of the cells for 15 min at room temperature serotonin was added to them up to final concentration of 10 µM and incubation was continued for 30 min. The newly formed cAMP was determined by reagent kit LANCE cAMP Kit, according to the method described by the kit manufacturer [G. Grynkiewicz, M. Poenie, R.Y. Tsien. J. Biol. Chem. 1985. V. 260. P. 3440-3450] (PerkinElmer, CIIIA). Table 6 shows the results of investigation of the disclosed compounds ability in concentration-dependent manner to block the functional response to serotonin stimulation of cells (HEK-293), steadily expressing recombinant human 5-HT₆ receptor. The data given testify that novel γ-carbolines of the general formula **1** are effective serotonin 5-HT₆ receptor antagonists. Some of γ-carbolines of the general formula **1** have IC₅₀ < 0.5 µM in the setting of cell function experiments (IC₅₀ - antagonist concentration blocking 5-HT₆ receptors for 50%).

**Table 6. Ability of γ-carbolines of the general formula 1 to block serotonin 5-HT₆ receptors in the setting of cell function experiments.**

| **N° comp.** | **Formula** | **IC₅₀, µM** |
|---|---|---|
| **1.1.1**(3)·HCl | 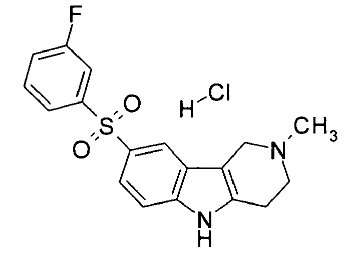 | 0.304 |
| **1.1**(1)·2HCl | 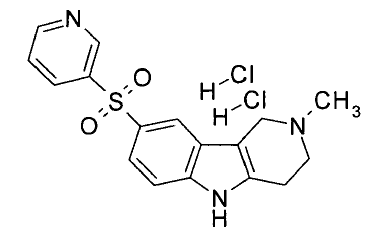 | 4.6 |
| **1.1**(2)·2HCl | 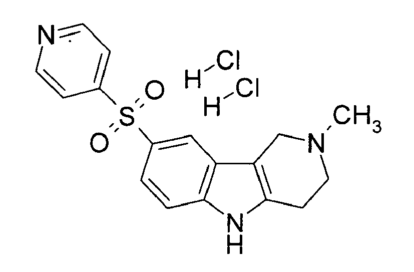 | 6.9 |
| **1.1.1**(4)·HCl | 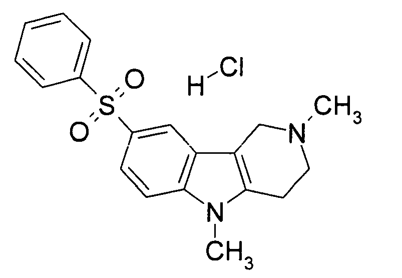 | 0.461 |
| **1.1**(10)·HCl | 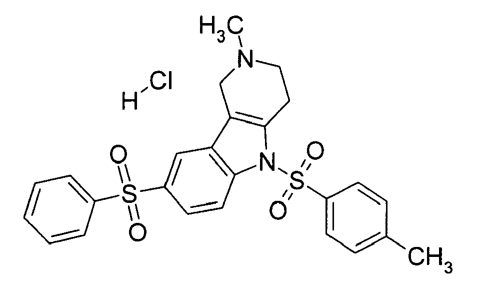 | ∼10 |
| **1.1**(16)·2HCl | 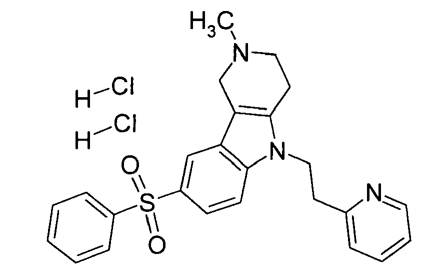 | 0.802 |
| **1.2**(1) | 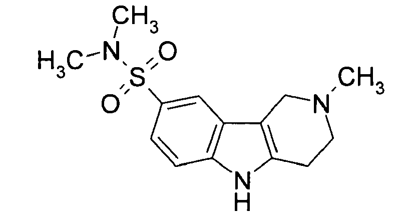 | ∼10 |
| **1.2**(6)·HCl | 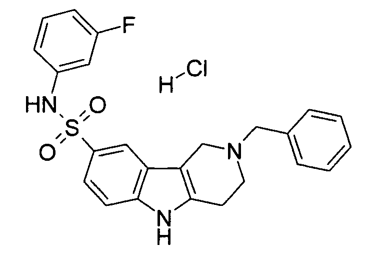 | 0.022 |
| **1.2.1**(3)·HCl | 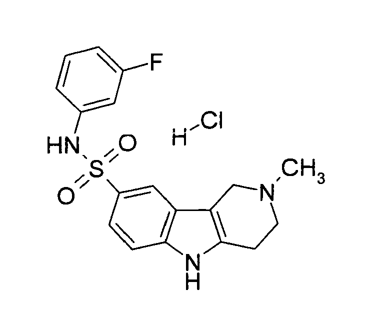 | 2.06 |
| **1.2**(10) | 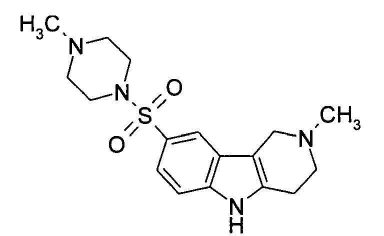 | >10 |

### Example 14. Investigation of pharmacological activity of substituted 8-sulfonyl-2,3,4,5-tetrahydro-1H-γ-earbolines of the general formula 1 towards various receptors.

The range of pharmacological activity of γ-carbolines of the general formula 1 towards various receptors was estimated by the value of the displacement of the corresponding receptors radio-labelled ligands by γ-carbolines at their concentration of 10 mkM. All radio-labelled experiments were carried out by MDS Pharma Services firm (http://www.mdsps.com/Services, Taipei, Taiwan) according to their protocols. Estimation of the results obtained is indicative of wide range of pharmacological activity of γ-carbolines of the general formula 1 towards alpha-adrenoceptors, dopamine receptors, histamine receptors, imidazoline receptors, sigma receptors, norepinephrine receptors and serotonin receptors. Figure 1 shows, as an example, the results for γ-carboline **1.1.1(4)·HCl** interaction with 31 receptors. As can be seen from fig. 1, γ-carboline **1.1.1(4)·HCl** actively interacts with 13 receptors (% of radio-labelled ligands displacement >60%). Analogous results were obtained for γ-carboline **1.2.1(3)·HCl** (Fig.2).

**Example 15**. Preparation of a medicament in the form of tablet. Starch (1600 mg), ground lactose (1600 mg), talk (400 mg) and 2,5-dimethyl-8-(phenylsulfonyl)-2,3,4,5-tetrahydro-1*H*-γ-carboline hydrochloride **1.1.1(4)·HCl** (1000 mg) or 2-methyl-2,3,4,5-tetrahydro-1*H*-γ-carboline-8-sulfonic acid 3-fluorophenylamide **1.2.1**(3)·HCl (1000 mg) were mixed together and pressed into bar. The resultant bar was comminuted into granules and sifted through sieve to collect granules of 14-16 mesh. The granules thus obtained were shaped into tablets of suitable form weighing 560 mg each. According to the invention medicaments comprising other ligands of the general formula 1 as active ingredients were prepared in the same manner.

**Example 16**. Preparation of a medicament in the form of capsules. Compound 2,5-dimethyl-8-(phenylsulfonyl)-2,3,4,5-tetrahydro-1*H*-γ-carboline hydrochloride **1.1.1**(4)·HCl or 2-methyl-2,3,4,5-tetrahydro-1*H*-γ-carboline-8-sulfonic acid 3-fluorophenylamide **1.2.1**(3)·HCl and lactose powder were carefully mixed in ratio 2 : 1. The resultant powdery mixture was packed into gelatin capsules of suitable size by 300 mg to a capsule.

**Example 17**. Preparation of medicament in the form of compositions for intramuscular, intraperitoneal or hypodermic injections. Compound 2,5-dimethyl-8-(phenylsulfonyl)-2,3,4,5-tetrahydro-1*H*-γ-carboline hydrochloride **1.1.1**(4)-HCl (500 mg) or 2-methyl-2,3,4,5-tetrahydro-1*H*-γ-carboline-8-sulfonic acid 3-fluorophenylamide **1.2.1**(3)·HCl (500 mg), chlorobutanol (300 mg), propylene glycol (2 ml), and injectable water (100 ml) were mixed together. The resultant solution was filtered and placed into 1 ml ampoules, and which were sealed and sterilized in an autoclave.

### Example 18. Anti-amnestic activity (nootropic action) of compounds of the general formula 1.

### 18.1. Enhancement of memory disturbed by Scopolamine.

18.1.1. Nootropic action of compounds of the general formula 1 in the test "Passive Avoidance of mice in the Shuttle Chamber". Experiments were carried out in aged male mice of BALB/c line weighing 20-25 grams or male rats of Vistar line weighing 200-250 g.

A shuttle chamber (Ugo Basile, Italy) consisted of two sections was used. The walls of one section were opaque, while the other section had a transparent cover. The sections were connected through a hole which could be overlapped by vertical door. The floor of the dark section was made of transverse metal bars on which DC current impulses could be fed. On the first day of experiment 30 minutes before training mice were injected intraintestinally with Scopolamine which causes memory disturbance. The animals of trial group were additionally injected with a compound of the general formula 1, for example, compound **1.1.1(4)HCl** or **1.2.1(3)HCl,** in doses of 0,2 mg/kg. The animals of control group were injected with physiological solution. Each group consisted of 8 animals. The animals were placed in the light section, and latent period of the first entry into the dark chamber was registered. Then the vertical door was closed and the animal was punished by 0.6 mA DC current for 3 seconds. After that the animal was taken back to its home cage. In 24 hours the same animal was placed again in the light section of the shuttle chamber and latent period of its first entry into the dark section, the total time of its stay in the light section and the number of entries into dark section was registered. Each monitoring lasted for 5 minutes.

Animals of the control group, having been punished in dark section, showed successful learning ability, which was expressed in prolongation of latent period of its entry into dark section, duration of its stay in light section and decreasing the number of entries into dark section in comparison with the group of animals which had not been punished. Scopolamine causes so-called anterograde amnesia, which is characterized by disfunction of new events fixation in long-term memory. It was expressed in the form of statistically significant prolongation of latent period of entry into dark section, decreasing the total time of stay in light section and increasing the number of entries into dark section.

The test results showed that in the test of "Passive Avoidance" compounds of the general formula 1, among them, compound **1.1.1(4)HCl,** and also compound **1.2.1(3)HCl** have the property to decrease amnesia (to enhance memory), caused by Scopolamine.

### 18.1.2. Nootropic action of compounds of the general formula 1 in "Novel object recognition" test.

The test was carried out in aged male mice of SHK line. Plexiglass plus maze, consisted of 4 dead-end chambers (numbered 1, 2, 3, 4), joined together through the fifth central chamber, was used in experiments. Mouse was placed into central chamber and allowed to explore the maze. Floor was cleaned after each animal. The sequence of chamber entries and duration of visits were registered by observer. Test was ended after 13 entries into dead-end chambers. Criterion for entry was location of all animal's paws inside the chamber at the same time.

During the training an animal was placed in the maze with one equiform bowl in each side chamber. In the course of testing (one hour after training) two opposite standing bowls were replaced by identical flasks, and animal was allowed to explore the maze. The time spent by animal in each side chamber was registered during the periods of training and testing. Index of novel object recognition was calculated as ratio of time spent by animal in side chambers with novel objects to the total time spent by it in all side chambers. In comparison with the training phase the appearance of novel object extends the time spent by animal in the chamber with novel object (so-called effect of novel object recognition). The recognition of novel objects was disturbed under action of Scopolamine administered in 1 mg/kg dose intraperitoneally 30 minutes before training, and recognition index was decreased. However, this influence of Scopolamine could be prevented by intraperitoneal administration of Dimebon (0,1 mg/kg) 5 minutes before training, Tacrine (10 mg/kg) 30 minutes before training and compounds **1.1.1(4)HCL** and **1.2.1(3) HCL** of the general formula 1 in doses of 0.05 mg/kg and 0.2 mg/kg 30 minutes before training.

The data obtained show that compounds of the general formula **1** prevent memory impairment caused by Scopolamine.

### 18.2. Enhancement of memory disturbed by MK-801.

The test was carried out as in example 18.1.1. On the first day of the test 30 minutes before training the animals were injected intraperitoneally with physiological solution of MK-801 (0.1 mg/kg) causing amnesia. Preliminary introduction of MK-801 considerably reduces training effect, in other words it caused anterograde amnesia. In parallel, 30 minutes before training MK-801 in combination with investigated compounds of the general formula 1 was injected intraperitoneally to independent groups of animals before training.

The test results show that compound **1.1.1(4)HCl** injected intraperitoneally before training in doses 0.2 mg/kg and 1 mg/kg, exhibit the property to decrease amnesia caused by MK-801 markedly. Compound **1.2.1(3)HCl** shows analogous effect.

**Example 19. Anxiolytic activity of compounds of the general formula 1 in "Mice Behavior in the Elevated Plus Maze" test**. The experiments were carried out in aged male mice of BALB/c line weighing about 25 g. Animals were housed 5-7 per cage with water and food available. None of animals was acquainted with experimental set-up before. Each experimental group included 8 animals.

The procedure used was described earlier by Lister (Lister R.G. The use of a plus-maze to measure anxiety in the mouse. Psychopharmacology, 1987; 92:180-185). Plexiglass set-up consisted of two opened arms of 30 x 5cm size and two closed arms of 30 x 5 x 15cm size was used. Side arms were closed with transparent plexiglass and were connected with central zone via platform of 5 x 5cm size. Opened arms, central platform and floor were made of black plexiglass. The set-up was mounted on a metallic base which was placed 38,5cm above floor level.

Animals were injected intraperitoneally with placebo, Buspirone (5 mg/kg, 30 minutes before training), Lorazepam (0.05 mg/kg, 60 minutes before training) or with one of the compounds of the general formula **1**, for example, compound **1.1.1(4)HCl** or compound **1.2.1(3)HCl** in doses 0.05 mg/kg and 0.2 mg/kg. Buspirone and Lorazepam were introduced in maximal effective dose, at which side sedative effect and general decrease of exploratory activity (number of arm entries during the test) were not observed yet.

Each mouse was placed in maze center with its head towards the opened arm. Over a period of 5 minutes the sequence and duration of arm entries were registered by means of computer program. Criterion for entry is location of all animal's paws inside the arm at the same time. Index of preference was calculated as ratio of the time spent by animal in opened arms as well as the number of entries into opened arms to the total time spent by it in opened and closed arms or, respectively, to the whole number of entries to arms of both types. The number of defecations left by mouse was regarded as additional parameter characterizing anxiety state.

The test results show that compounds of the general formula 1, among them compounds **1.1.1(4)HCl** and **1.2.1(3)HCl,** as well as standard compounds (Buspirone and Lorazepam), produced well-marked anxiolytic effect in test "Mice Behavior in the Elevated Plus Maze".

**Example 20**. Antipsychotic activity of compounds of the general formula 1 in "Prepulse inhibition of the startle response in mice" test. Mice of SHK line weighing about 24-30g were used in the test. Experiments were carried out during light period of animal's diurnal. Apomorphine hydrochloride and Haloperidol were received from Sigma Chemicals Company, (USA). Apomorphine hydrochloride was dissolved in 0.1 % solution of ascorbic acid prepared with sterilized water; it was introduced subcutaneously 15 minutes before the test. Haloperidol was dissolved in sterilized water using emulsifier Twin 80, it was introduced intraperitoneally 60 minutes before the test. Compounds of the general formula 1 were dissolved in sterilized water, they were introduced subcutaneously 60 minutes before the test. Injection volume was 10 ml/kg. Solution of ascorbic acid, prepared with sterilized water and Twin 80, were injected to control group of animals.

The test instrument consisted of a chamber made of transparent plexiglass (manufacturer - Columbia Instruments Company, USA) and placed on a platform; the latter was lodged inside the sound insulating chamber. High frequency sound colomn transmitting acoustic stimuluses was located 2 cm away from the platform. Startle of animal resulted in vibrations of platform, which were detected by analog converter and registered by computer. Level of background noise made up 65 dB. Each animal received 4 stimuli of single testing (pulse) stimulus of 50 ms duration and 105 dB or prepulsory stimulus (pre-pulse) of 20 ms duration and 85 dB, after which in 30 ms pulse stimulus of 50 ms duration and 105 dB followed. Time interval between repeated pulse or prepulse in combination with pulse stimuli made up 10 s. Inhibition of the startle in reply to prepulse-plus-pulse stimulus was calculated in percentage towards amplitude of startle in response to isolated pulse stimulus. Administration of Apomorphine, which is used in experiments on animals for modelling of psychoto-like conditions, caused reduction of prepulse inhibition of startle, which reflected the lowering of CNS ability to filter sensory stimulus.

The results of the experiment show that Haloperidol (1mg/kg) and the tested compounds **1.1.1(4)HCl** and **1.2.1(3)HCl** (1mg/kg) of the general formula 1, prevented disturbance of prepulse inhibition of startle caused by Apomorphine.

**Example 21**. Mice Training in the Morris Water maze. A round pool filled with water at 20-22°C was used. There was a round ceramic platform of 14 cm height in the pool. Animals's (mice) behavior was registered with an automated computer video system in combination with software package of movement analysis Any-maze (Stoelting Co., CIIIA). Selection of mice suitable for training was carried out before the experiments. For this purpose the platform was placed 1 cm above the water level and an animal was put on the platform for 20 seconds. Then the mouse was sunk into the water on the opposite side of the pool, allowed to find the platform and climb it for 60 seconds, where it was left for 20 seconds. After that the mouse was repeatedly immersed into water on the opposite side of the pool and allowed to find the platform. If it failed in finding the platform within 60 seconds the experimentator helped it to find the platform and climb it. If the mice couldn't find the platform itself in two consecutive attempts it was excluded from the experiment.

During the next two days the platform was placed 0.5 cm lower the water level. Every day the mice were given four attempts for finding the platform within 60 seconds. The time interval between the attempts was 20 seconds, during which the mice stayed on the platform. Every day before the first attempt the mice was placed on the platform for 20 seconds. The time needed for finding and climbing the platform was registered. The animals were sunk into water in three different places on the side of the pool opposite to the platform. On each day of two-days' experiment 35-40 minutes before training the animals were injected intraperitoneally with saline solution, Scopolamine or Scopolamine in combination with a tested compound. At least 8 animals were used in each group.

On the third day the platform was removed and animals were placed once into the pool for 60 seconds. The time each mouse spent in the area where the platform had been located during the previous days was registered. This time interval was regarded as a measure of learning efficiency carried out during the previous two days.

Experiments were carried out during light period of animal's diurnal in isolated laboratory room, level of white noise made up 70 dB above normal threshold of audibility. Test results for, compounds **1.1.1(4)HCl, 1.2.1(3)HCl** testify decreasing of memory impairment caused by Scopolamine after single-dose treatment with medicinal substance. This fact supports their therapeutic effect at neurodegenerative diseases and cognitive disorders, among them Alzheimer's disease.

**Example 22.** Mice Behavior in Porsolt's Forced Swim Test. The test apparatus represented a plastic vessel filled with water up to height of 18 cm at 20-22°C. Mice were placed in water and for 15 minutes the duration of immobile hanging in water - so called behavioural despaire, which is considered to be a measure of depressively-like state, was registered. Automated computerized detection of motion with videosystem and Any-maze programm were used in test. After ligands **1.1.1(4)HCl** and **1.2.1(3)HCl** were injected for 4 days in doses of 1 mg/kg the duration of immobile hanging of mice in water was decreased almost thrice. This testifies the decreasing of time of a depressively-like state.

**Example 23**. Mice behavior in tail suspension test. Mice were suspended by tail with scotch tape on holder over horizontal surface at a height of about 40cm, and for 6 minutes the total duration of complete immobility episodes which is considered to be a measure of depressively-like state was registered. Automated computerized detection of motion with videosystem and Any-maze programm were used in test. After ligands **1.1.1(4)HCl** and **1.2.1(3)HCl** were injected for 4 days in doses of 0.05 mg/kg the duration of mice complete immobility was decreased significantly. This testifies the decreasing of time of a depressively-like state.

### Industrial applicability

The invention could be used in medicine, veterinary, biochemistry.

## Claims

1. Substituted 8-sulfonyl-2,3,4,5-tetrahydro-1*H*-γ-carbolines of the general formula 1 and pharmaceutically acceptable salts thereof, 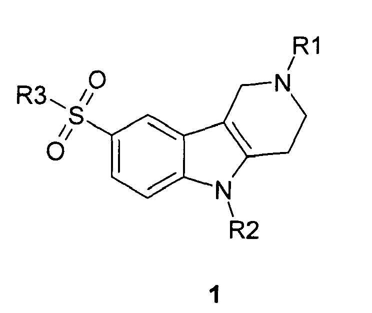 wherein
**R¹** represents a substituent selected from hydrogen; C₁-C₃ alkyl, optionally substituted with phenyl; C₁-C₄ alkoxycarbonyl;
**R²** represents a substituent of cyclic system selected from hydrogen; C₁-C₃ alkyl, optionally substituted with phenyl, pyridin-(3- or 4-yl), (6-methylpyridin-3-yl); C₂-C₃ alkenyl, substituted with phenyl; optionally substituted phenylsulfonyl;
**R³** represents phenyl, optionally substituted with halogen; 6-membered aromatic azaheterocyclyl; C₁-C₃ dialkylamino group; phenylamino group, in which the phenyl ring is optionally substituted with halogen; saturated 6-membered azaheterocyclyl comprising an additional nitrogen atom, substituted with C₁-C₃ alkyl.

2. Compounds according to claim 1, representing 8-arylsulfonyl-2,3,4,5-tetrahydro-1*H* γ-carbolines of the general formula **1.1** and amides of 2,3,4,5-tetrahydro-1*H*-γ-carboline-8-sulfonic acid of the general formula **1.2** and pharmaceutically acceptable salts thereof, 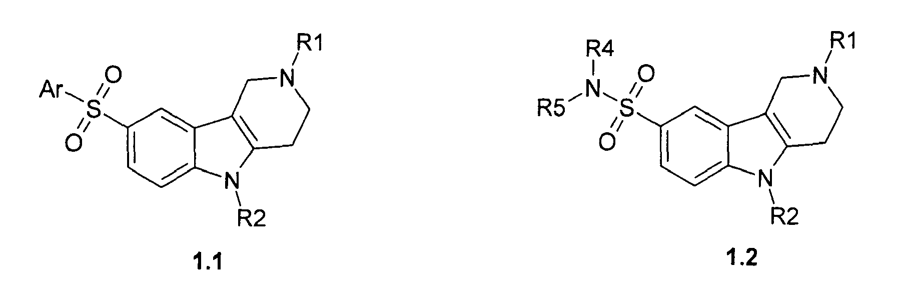 wherein:
**R¹** and **R²** have the above meanings;
**Ar** represents phenyl, optionally substituted with halogen, or a 3- or 4-pyridyl,
**R⁴** and **R⁵** are optionally identical substituents representing hydrogen; optionally substituted C₁-C₃ alkyl; phenyl optionally substituted with halogen, with the proviso that **R⁴** and **R⁵** may not be simultaneously hydrogen; or **R⁴** and **R⁵** together with the nitrogen atom to which they are attached form 4-alkylsubstituted piperazin-1-yl.

3. Compounds according to claim 2, representing substituted 8-phenylsulfonyl-2,3,4,5-tetrahydro-1*H*-γ-carbolines of the general formula **1.1.1** and substituted phenylamides of 2,3,4,5-tetrahydro-1*H*-γ-carbolin-8-sulfonic acid of the general formula **1.2.1** and pharmaceutically acceptable salts thereof, 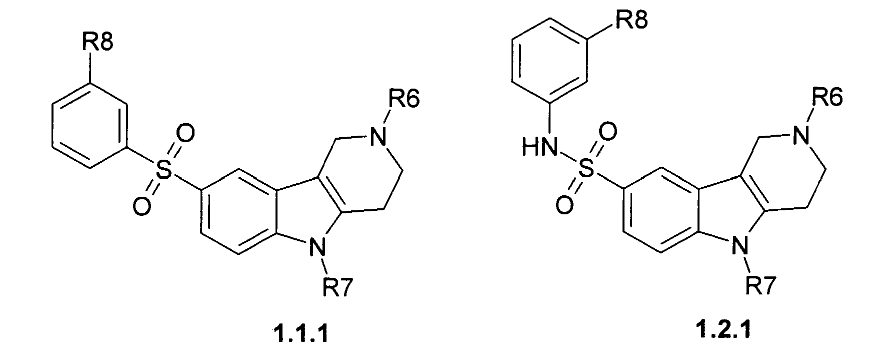 wherein:
**R⁶** and **R⁷** independently of each other represent hydrogen or methyl;
**R⁸** represents hydrogen, chloro or fluoro.

4. Compounds according to claim 3 representing: 2-methyl-8-phenylsulfonyl-2,3,4,5-tetrahydro-1*H*-γ-carboline **1.1.1**(1), 2-methyl-8-(3-chlorophenyl)sulfonyl-2,3,4,5-tetrahydro-1*H*-γ-carboline **1.1.1**(2), 2-methyl-8-(3-fluorophenyl)sulfonyl-2,3,4,5-tetrahydro-1*H*-γ-carboline **1.1.1**(3), 2,5-dimethyl-8-phenylsulfonyl-2,3,4,5-tetrahydro-1*H*-γ-carboline **1.1.1**(4), 2,5-dimethyl-8-(3-chlorophenyl)sulfonyl-2,3,4,5-tetrahydro-1*H*-γ-carboline **1.1.1**(5), 2,5-dimethyl-8-(3-fluorophenyl)sulfonyl-2,3,4,5-tetrahydro-1*H*-γ-carboline **1.1.1**(6), 5-methyl-8-phenylsulfonyl-2,3,4,5-tetrahydro-1*H*-γ-carboline **1.1.1**(7), 5-methyl-8-(3-chlorophenyl)sulfonyl-2,3,4,5-tetrahydro-1*H*-γ-carboline **1.1.1**(8), 5-methyl-8-(3-fluorophenyl)sulfonyl-2,3,4,5-tetrahydro-1*H*-γ-carboline **1.1.1**(9), 2-methyl-2,3,4,5- tetrahydro-1*H*-γ-carboline-8-sulfonic acid phenylamide **1.2.1**(1), 2-methyl-2,3,4,5-tetrahydro-1*H*-γ-carboline-8-sulfonic acid 3-chlorophenylamide **1.2.1**(2), 2-methyl-2,3,4,5-tetrahydro-1*H*-γ-carboline-8-sulfonic acid 3-fluorophenylamide **1.2.1**(3), 2,5-dimethyl-2,3,4,5-tetrahydro-1*H*-γ-carboline-8-sulfonic acid phenylamide **1.2.1**(4), 2,5-dimethyl-2,3,4,5-tetrahydro-1*H*-γ-carboline-8-sulfonic acid 3-chlorophenylamide **1.2.1**(5), 2,5-dimethyl-2,3,4,5-tetrahydro-1*H*-γ-carboline-8-sulfonic acid 3-fluorophenylamide **1.2.1**(6), 5-methyl-2,3,4,5-tetrahydro-1*H*-γ-carboline-8-sulfonic acid phenylamide **1.2.1**(7), 5-methyl-2,3,4,5-tetrahydro-1*H*-γ-carboline-8-sulfonic acid 3-chlorophenylamide **1.2.1**(8), 5-methyl-2,3,4,5-tetrahydro-1*H*-γ-carboline-8-sulfonic acid 3-fluorophenylamide **1.2.1**(9) and pharmaceutically acceptable salts thereof,

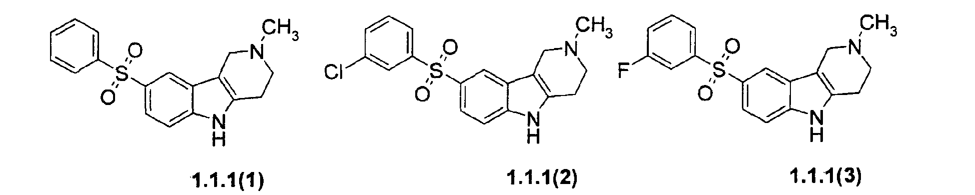

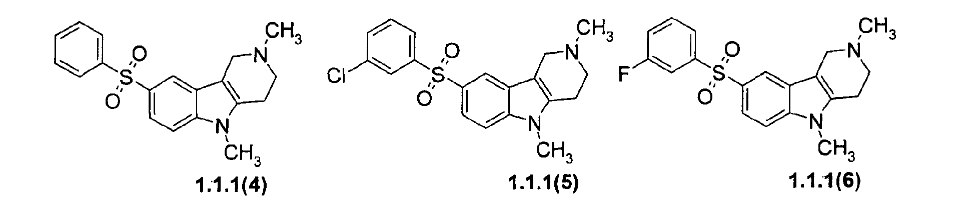

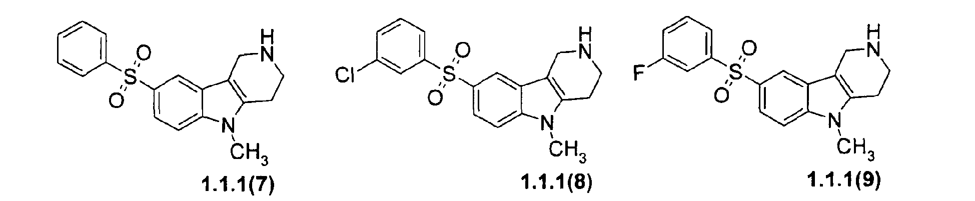

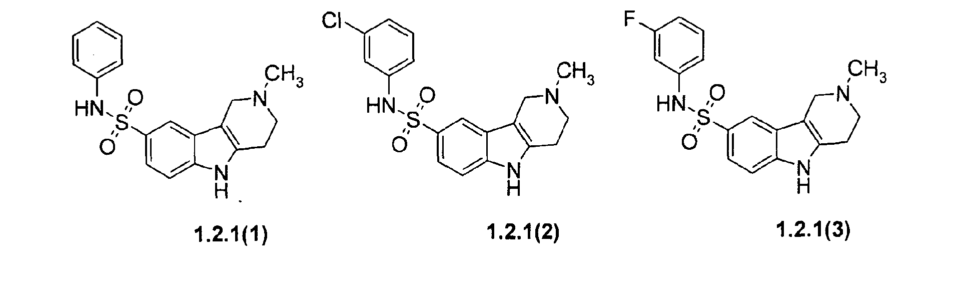

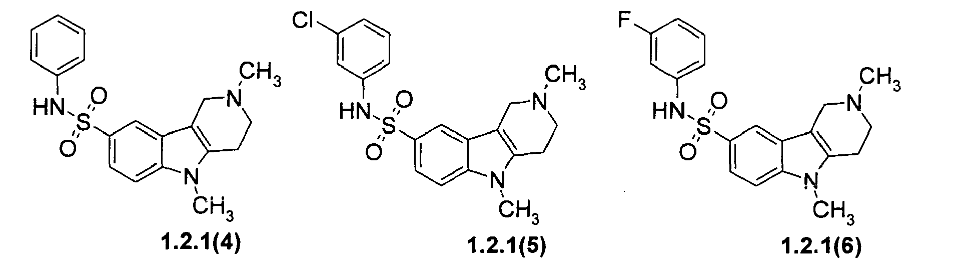

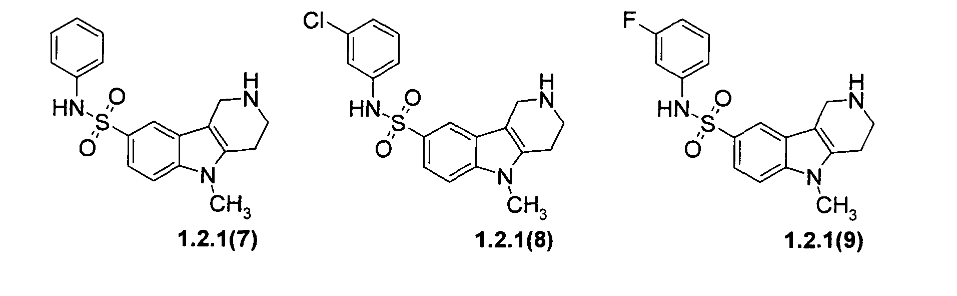

5. Method for the preparation of 8-arylsulfonyl-2,3,4,5-tetrahydro-1*H*-γ-carbolines of the general formula **1.1** according to claim 2 by interaction of 8-bromo-2,3,4,5-tetrahydro-1*H*-γ-carbolines of the general formula 2 with sulfinic acids of the general formula 3 in the presence of cuprous iodide, N,N'-dimethylethylenediamine and a base in an aprotonic solvent: 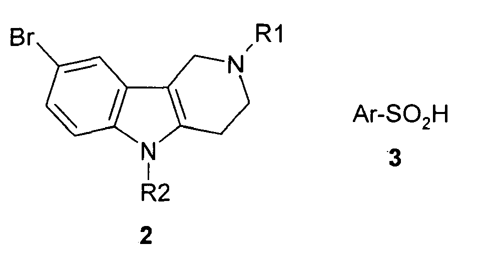 wherein:
**R¹, R²** and **Ar** have the above meanings.

6. Method for the preparation of 8-arylsulfonyl-2,3,4,5-tetrahydro-1*H*-γ-carbolines of the general formula 1.1 according to claim 2 by interaction of 2,3,4,5-tetrahydro-1*H*-γ-carboline-8-sulfinic acid of the general formula 4 with aryl iodides of the general formula 5 in the presence of cuprous iodide, N,N'-dimethylethylenediamine and a base in an aprotonic solvent: 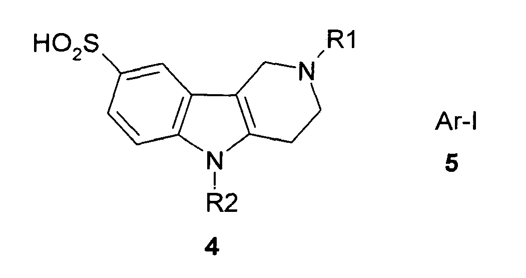 wherein:
**R¹, R²** and **Ar** have the above meanings.

7. Method for the preparation of 2,3,4,5-tetrahydro-1*H*-γ-carboline-8-sulfonic acid amides of the general formula 1.2 according to claim 2 by interaction of 2,3,4,5-tetrahydro-1*H* γ-carboline-8-sulfonyl chlorides of the general formula 6 with amines of formula 7 in the presence of a base, 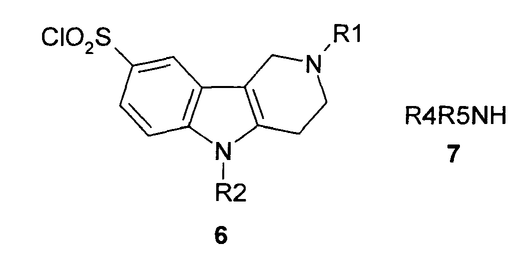 wherein:
**R¹, R², R⁴** and **R⁵** have the above meanings.

8. Ligands the spectrum of biological activity of which simultaneously comprises alpha-adrenoceptors, dopamine receptors, histamine receptors, imidazoline receptors, sigma receptors, norepinefrine receptors, serotonin receptors, representing substituted 8-sulfonyl-2,3,4,5-tetrahydro-1*H*-γ-carbolines of the general formula 1 according to claim 1 in the form of free bases or pharmaceutically acceptable salts.

9. Ligands according to claim 8 exhibiting antagonistic activity with respect to serotonin 5-HT₆ receptors.

10. Ligand of the general formula 1 according to claims 8 and 9 in the form of free bases or pharmaceutically acceptable salts as an active component for pharmaceutical compositions and medicaments intended for the treatment and prophylaxis of pathologic conditions and diseases of the CNS.

11. Pharmaceutical composition exhibiting biological activity simultaneously with respect to alpha-adrenoceptors, dopamine receptors, histamine receptors, imidazoline receptors, sigma receptors, norepinefrine receptors, serotonin receptors, comprising as an active component a therapeutically effective amount of a ligand of the general formula 1 according to claim 10 or pharmaceutically acceptable salts thereof.

12. Method for the preparation of a pharmaceutical composition according to claim 11 consisting in mixing of at least one ligand according to claim 10 with an inert filler and/or solvent.

13. A medicament exhibiting biological activity with respect to alpha-adrenoceptors, dopamine receptors, histamine receptors, imidazoline receptors, sigma receptors, norepinefrine receptors, serotonin receptors intended for the treatment and prophylaxis of pathological conditions and diseases of the CNS in the form of tablets, capsules or injections, placed in a pharmaceutically acceptable packing comprising a ligand according to claim 10 or a pharmaceutical composition according to claim 11.

14. Method for the treatment and/or prophylaxis of diseases and pathological conditions of the CNS by administration of a therapeutically effective amount of an active component according to claim 10, or a pharmaceutical composition according to claim 11, or a medicament according to claim 13.

15. The method according to claim 14 for the treatment and/or prophylaxis of anxiety disorders.

16. The method according to claim 14 for the treatment and/or prophylaxis of cognitive disorders and neurodegenerative diseases.

17. The method according to claim 14 for the treatment and/or prophylaxis of psychotic diseases.

18. The method according to claim 14 for the treatment and/or prophylaxis of depression.
